# EUROPEAN PATENT APPLICATION

(11) **EP 1 980 564 A1**
(43) Date of publication of application: **15.10.2008**
(21) Application number: 08155302.6
(22) Date of filing: 18.12.2003
(51) Int. Cl.: C07F 5/02, A61K 31/47, A61K 31/04, A61P 31/10

(54) **Antibiotics containing borinic acid complexes and methods of use**

(30) Priority: 18.12.2002 US 434375; 23.12.2002 US 436095; 03.01.2003 US 437849
(62) Divisional of application: 03800095.6
(71) Applicant: Anacor Pharmaceuticals, Inc., Palo Alto, CA 94303 (US)
(72) Inventor: Lee, Ving, LOS ALTOS, PA 94024 (US); Benkovic, Stephen J., STATE COLLEGE, PA 16803 (US)
(74) Representative: Couchman, Jonathan Hugh

(57) **Abstract**

The structure and preparation of antibiotics incorporating borinic acid complexes are disclosed, especially hydroxyquinoline, imidazole and picolinic acid derivatives, along with compositions of these antibiotics and methods of using the antibiotics and compositions as bactericidal and fungicidal agents as well as therapeutic agents for the treatment of diseases caused by bacteria and fungi.

## Description

This application claims priority of U.S. Provisional Application Serial No. 60/434,375, filed 18 December 2002, Serial No. 60/436,095, filed 23 December 2002, and Serial No. 60/437,849, filed 3 January 2003, the disclosures of which are hereby incorporated by reference in their entirety.

### FIELD OF THE INVENTION

The present invention relates to the field of antibiotics and particularly antibacterial and antifungal compounds and uses thereof. Methods for preparing and using these antibiotics, and pharmaceutical compositions thereof, are also provided.

### BACKGROUND OF THE INVENTION

One hallmark of the modern era of medicine has been the decline in morbidity and mortality associated with bacterial and fungal infections. However, misuse of conventional antibiotics and natural selection of the infectious bacterial population has resulted in the development of varying degrees of drug resistance by most bacterial infectious agents to most antibiotic agents. In severe cases, such as MRSA (Multidrug-Resistant StaphA), one or only a few antibiotics are currently effective. In addition, the existence of immunodeficiency syndromes results in additional incidence of opportunistic infections requiring intensive antibiotic treatment.

Thus, there continues to be a need in the medical arts for novel, more effective, antibiotic compounds, especially for treating bacterial infections, that are resistant to currently available therapies.

### BRIEF SUMMARY OF THE INVENTION

In one aspect, the present invention relates to antibiotic compounds. The antibiotic compounds of the invention are borinate derivatives, especially borinic acid complexes, and include such compounds as derivatives of hydroxyquinolines, picolinic acids and imidazoles.

The antibiotic compounds are also provided as pharmaceutical compositions that can be administered to an animal, most preferably a human, for treatment of a disease having a bacterial or fungal etiology, or an opportunistic infection with a bacteria or fungus in an animal, most preferably a human, in an immunologically compromised or debilitated state of health.

In preferred embodiments, the compounds of the invention are those having the structures given by Formulas 1 or 2, with preferred substituents as disclosed herein.

The invention also provides methods for preparing the antibiotic compounds and pharmaceutical compositions thereof, and methods of using said antibiotics therapeutically. Kits and packaged embodiments of the antibiotic compounds and pharmaceutical compositions of the invention are also contemplated.

The invention also relates to methods of treating infections, preferably bacterial and/or fungal infections, using the antibiotic compounds disclosed herein.

### DETAILED DESCRIPTION OF THE INVENTION

This invention provides antibiotics, and specifically antibacterial and anti-fungal compounds, useful in treating and/or preventing bacterial infections.

The invention comprises a compound having the structure with Formula 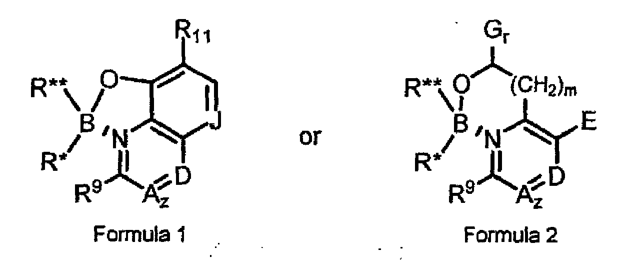 wherein B is boron, O is oxygen, m is 0, 1, or 2,
wherein R* and R** are each independently selected from substituted or unsubstituted alkyl (C₁ - C₄), substituted or unsubstituted cycloalkyl (C₃ - C₆), substituted or unsubstituted vinyl, substituted or unsubstituted alkynyl, substituted or unsubstituted benzyl, substituted or unsubstituted phenyl, and substituted or unsubstituted heterocycle,
and wherein z is 0 or 1 and when z is 1, A is CH, CR¹⁰ or N,
and wherein D is N, CH, or CR¹²,
and wherein E is H, OH, alkoxy or N-(morpholinyl)ethoxy
and wherein r is 1 or 2, and wherein when r is 1, G is =O (double-bonded oxygen) and when r is 2, each G is independently H, methyl, ethyl or propyl,
wherein R¹² is selected from (CH₂)ₖOH (where k = 1, 2 or 3), CH₂NH₂, CH₂NH-alkyl, CH₂N(alkyl)₂, CO₂H, CO₂alkyl, CONH₂, OH, alkoxy, aryloxy, SH, S-alkyl, S-aryl, SO₂alkyl, SO₃H, SCF₃, CN, halogen, CF₃, NO₂, NH₂, 2*-amino, 3*-amino, NH₂SO₂ and CONH₂,
and wherein J is CR¹⁰ or N
and wherein R⁹, R¹⁰ and R¹¹ are each independently selected from the group consisting of hydrogen, alkyl, (CH₂)ₙOH (n = 1 to 3), CH₂NH₂, CH₂NHalkyl, CH₂N(alkyl)₂, halogen, CHO, CH=NOH, CO₂H, CO₂-alkyl, S-alkyl, SO₂-alkyl, S-aryl, NH₂, alkoxy, CF₃, SCF₃, NO₂, SO₃H and OH,
including salts thereof, especially all pharmaceutically acceptable salts.

In a preferred embodiment of either of Formulas 1 or 2, one of R* and R** is a substituted or unsubstituted alkyl (C₁ - C₄) or R* and R** are each a substituted or unsubstituted alkyl (C₁ - C₄).

In a preferred embodiment of either of Formulas 1 or 2, one of R* and R** is a substituted or unsubstituted cycloalkyl (C₃ - C₆) or R* and R** are each a substituted or unsubstituted cycloalkyl (C₃ - C₆).

In a preferred embodiment of either of Formulas 1 or 2, one of R* and R** is a substituted or unsubstituted vinyl or R* and R** are each a substituted or unsubstituted vinyl. In a further preferred embodiment thereof, the vinyl has the structure 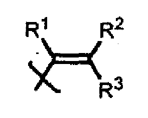 wherein R¹, R², and R³ are each independently selected from the group consisting of hydrogen, alkyl, aryl, substituted aryl, benzyl, substituted benzyl, (CH₂)ₖOH (where k = 1, 2 or 3), CH₂NH₂, CH₂NH-alkyl, CH₂N(alkyl)₂, CO₂H, CO₂alkyl, CONH₂, S-alkyl, S-aryl, SO₂alkyl, SO₃H, SCF₃, CN, halogen, CF₃ and NO₂.

In a preferred embodiment of either of Formulas 1 or 2, one of R* and R** is a substituted or unsubstituted alkynyl or R* and R** are each a substituted or unsubstituted alkynyl. In a further preferred embodiment thereof the alkynyl has the structure 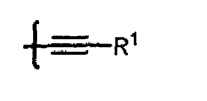 wherein R¹ is selected from the group consisting of hydrogen, alkyl, aryl, substituted aryl, benzyl, substituted benzyl, (CH₂)ₖOH (where k = 1, 2 or 3), CH₂NH₂, CH₂NH-alkyl, CH₂N(alkyl)₂, CO₂H, CO₂alkyl, CONH₂, S-alkyl, S-aryl, SO₂alkyl, SO₃H, SCF₃, CN, halogen, CF₃ and NO₂.

In a preferred embodiment of either of Formulas 1 or 2, one of R* and R** is a substituted or unsubstituted phenyl or R* and R** are each a substituted or unsubstituted phenyl. In a further preferred embodiment thereof the phenyl has the structure 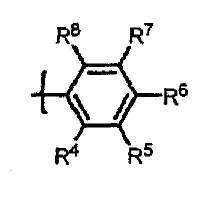 wherein R⁴, R⁵, R⁶, R⁷ and R⁸ are each independently selected from the group consisting of hydrogen, alkyl, aryl, substituted aryl, benzyl, substituted benzyl, (CH₂)ₖOH (where k = 1, 2 or 3), CH₂NH₂, CH₂NH-alkyl, CH₂N(alkyl)₂, CO₂H, CO₂alkyl, CONH₂, CONHalkyl, CON(alkyl)₂, OH, alkoxy, aryloxy, SH, S-alkyl, S-aryl, SO₂alkyl, SO₃H, SCF₃, CN, halogen, CF₃, NO₂, NH₂, 2°-amino, 3°-amino, NH₂SO₂, OCH₂CH₂NH₂, OCH₂CH₂NHalkyl, OCH₂CH₂N(alkyl)₂, oxazolidin-2-yl, or alkyl substituted oxazolidin-2-yl.

In a preferred embodiment of either of Formulas 1 or 2, one of R* and R** is a substituted or unsubstituted benzyl or R* and R** are each a substituted or unsubstituted benzyl. In a further preferred embodiment thereof the benzyl has the structure 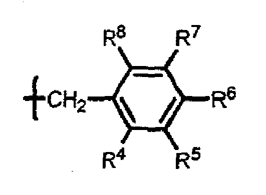 wherein R⁴, R⁵, R⁶, R⁷ and R⁸ are each independently selected from the group consisting of alkyl, aryl, substituted aryl, benzyl, substituted benzyl, (CH₂)ₖOH (where k = 1, 2 or 3), CH₂NH₂, CH₂NH-alkyl, CH₂N(alkyl)₂, CO₂H, CO₂alkyl, CONH₂, CONHalkyl, CON(alkyl)₂, OH, alkoxy, aryloxy, SH, S-alkyl, S-aryl, SO₂alkyl, SO₃H, SCF₃, CN, halogen, CF₃, NO₂, NH₂, 2°-amino, 3°-amino, NH₂SO₂, OCH₂CH₂NH₂, OCH₂CH₂NHalkyl, OCH₂CH₂N(alkyl)₂, oxazolidin-2-yl, or alkyl substituted oxazolidin-2-yl..

In a preferred embodiment of either of Formulas 1 or 2, one of R* and R** is a substituted or unsubstituted heterocycle or R* and R** are each a substituted or unsubstituted heterocycle. In a further preferred embodiment thereof the heterocycle has the structure 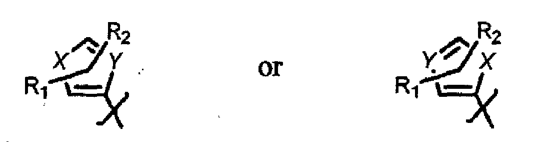 wherein X = CH=CH, N=CH, NR¹³ (wherein R¹³ = H, alkyl, aryl or benzyl), O, or S
and wherein Y = CH or N
and wherein R¹, R², and R³ are each independently selected from the group consisting of hydrogen, alkyl, aryl, substituted aryl, benzyl, substituted benzyl, (CH₂)ₖOH (where k = 1, 2 or 3), CH₂NH₂, CH₂NH-alkyl, CH₂N(alkyl)₂, CO₂H, CO₂alkyl, CONH₂, S-alkyl, S-aryl, SO₂alkyl, SO₃H, SCF₃, CN, halogen, CF₃ and NO₂.

The structures of the invention also permit solvent interactions that may afford structures (Formulas 1B and 2B) that include atoms derived from the solvent encountered by the compounds of the invention during synthetic procedures and therapeutic uses. Thus, such solvent structures can especially insinuate themselves into the compounds of the invention between the boron and nitrogen atoms, thereby affording a ring size one or two atom larger than that discloses in the structures herein. For example, where the boron ring of a structure of the invention comprises 5 atoms, including, for example, the boron, a nitrogen, an oxygen and 2 carbons, insinuation of a solvent atom between the boron and nitrogen would afford a 7 membered ring. In one example, use of hydroxyl and amino solvents may afford structures containing an oxygen or nitrogen between the ring boron and nitrogen atoms to increase the size of the ring. Such structures are expressly contemplated by the present invention where R*** is H or alkyl 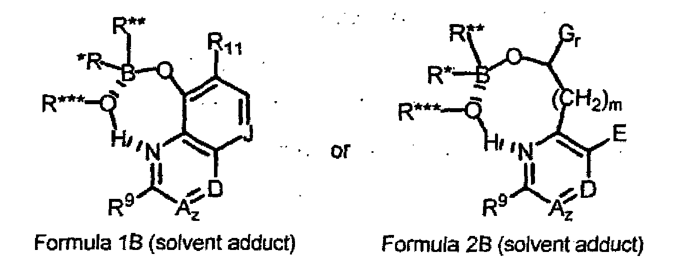 As used herein, the following terms have the stated meaning:

By "alkyl", "lower alkyl", and "C₁-C₆ alkyl" in the present invention is meant straight or branched chain alkyl groups having 1-6 carbon atoms, such as, methyl, ethyl, propyl, isopropyl, *n*-butyl, *sec-*butyl, *tert*-butyl, pentyl, 2-pentyl, isopentyl, neopentyl, hexyl, 2-hexyl, 3-hexyl, and 3-methylpentyl.

By "alkoxy", "lower alkoxy", and "C₁-C₆ alkoxy" in the present invention is meant straight or branched chain alkoxy groups having 1-6 carbon atoms, such as, for example, methoxy, ethoxy, propoxy, isopropoxy, *n*-butoxy, *sec-*butoxy, *tert*-butoxy, pentoxy, 2-pentyl, isopentoxy, neopentoxy, hexoxy, 2-hexoxy, 3-hexoxy, and 3-methylpentoxy.

By the term "halogen" in the present invention is meant fluorine, bromine, chlorine, and iodine.

By "cycloalkyl", e.g., C₃-C₇ cycloalkyl, in the present invention is meant cycloalkyl groups having 3-7 atoms such as, for example cyclopropyl, cyclobutyl, cyclopentyl, cyclohexyl, and cycloheptyl. In the C₃-C₇ cycloalkyl groups, preferably in the C₅-C₇ cycloalkyl groups, one or two of the carbon atoms forming the ring can optionally be replaced with a hetero atom, such as sulfur, oxygen or nitrogen. Examples of such groups are piperidinyl, piperazinyl, morpholinyl, pyrrolidinyl, imidazolidinyl, oxazolidinyl, perhydroazepinyl, perhydrooxazapinyl, oxepanyl, and perhydrooxepanyl. C₃ and C₄ cycloalkyl groups having a member replaced by nitrogen or oxygen include aziridinyl, azetidinyl, oxetanyl, and oxiranyl.

By "aryl" is meant an aromatic carbocyclic group having a single ring (e.g., phenyl), multiple rings (e.g., biphenyl), or multiple condensed rings in which at least one is aromatic, (e.g., 1,2,3,4-tetrahydronaphthyl, naphthyl, anthryl, or phenanthryl), which is optionally mono-, di-, or trisubstituted with, e.g., halogen, lower alkyl, lower alkoxy, lower alkylthio, trifluoromethyl, lower acyloxy, aryl, heteroaryl, and hydroxy. Preferred aryl groups include phenyl and naphthyl, each of which is optionally substituted as defined herein.

By "heteroaryl" is meant one or more aromatic ring systems of 5-, 6-, or 7-membered rings containing at least one and up to four heteroatoms selected from nitrogen, oxygen, or sulfur. Such heteroaryl groups includes, for example, thienyl, furanyl, thiazolyl, imidazolyl, (is)oxazolyl, pyridyl, pyrimidinyl, (iso)quinolinyl, napthyridinyl, benzimidazolyl, and benzoxazolyl. Preferred heteroaryls are thiazolyl, pyrimidinyl, preferably pyrimidin-2-yl, and pyridyl. Other preferred heteroaryl groups include 1-imidazolyl, 2-thienyl, 1-, or 2-quinolinyl, 1-, or 2- isoquinolinyl, 1-, or 2-tetrahydroisoquinolinyl, 2- or 3- furanyl and 2- tetrahydro-furanyl.

By "ligand" is meant a nitrogen-containing aromatic system which is capable of forming a dative bond with the Lewis acidic boron center, while appended as a borinate ester moiety. Such ligands are known to those trained in the arts. Examples are shown in the structures below.

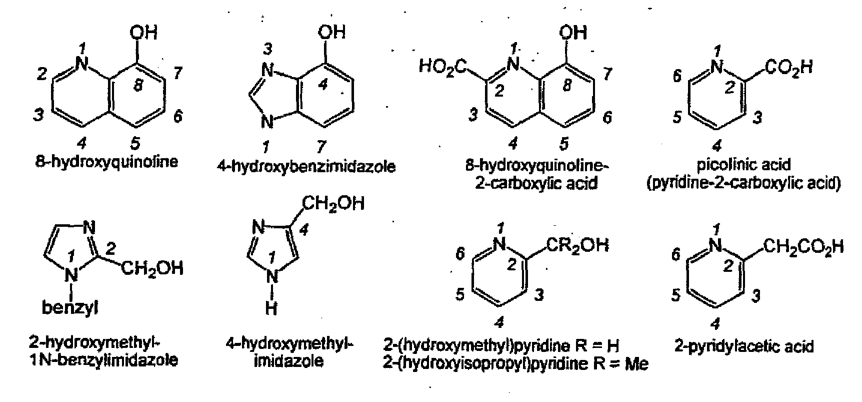

The compounds of the present invention have been implicated in the inhibition of key microbial enzymes, such as bacterial DNA methyltransferase. Many of the compounds disclosed herein are selective inhibitors of methyltransferases in microbes, while not inhibitory for methyltransferases in mammals. However, the anti-bacterial and anti-fungal activity of the compounds of the invention is not limited to those with said enzyme inhibitory activity, nor is the latter effect necessarily essential to said therapeutic activity.

The invention also provides embodiments of the compounds disclosed herein as pharmaceutical compositions. The pharmaceutical compositions of the present invention can be manufactured in a manner that is itself known, *e.g.*, by means of a conventional mixing, dissolving, granulating, dragee-making, levigating, emulsifying, encapsulating, entrapping or lyophilizing processes.

Pharmaceutical compositions for use in accordance with the present invention thus can be formulated in conventional manner using one or more physiologically acceptable carriers comprising excipients and auxiliaries that facilitate processing of the active compounds into preparations that can be used pharmaceutically. Proper formulation is dependent upon the route of administration chosen.

Non-toxic pharmaceutical salts include salts of acids such as hydrochloric, phosphoric, hydrobromic, sulfuric, sulfinic, formic, toluenesulfonic, methanesulfonic, hydroxyethanesulfonic, nitric, benzoic, citric, tartaric, maleic, hydroiodic, alkanoic such as acetic, HOOC-(CH₂)ₙ-CH₃ where n is 0-4, and the like. Non-toxic pharmaceutical base addition salts include salts of bases such as sodium, potassium, calcium, ammonium, and functional equivalents. Those skilled in the art will recognize a wide variety of non-toxic Pharmaceutical acceptable addition salts.

For injection, the compounds of the invention can be formulated in appropriate aqueous solutions, such as physiologically compatible buffers such as Hanks's solution, Ringer's solution, or physiological saline buffer. For transmucosal and transcutaneous administration, penetrants appropriate to the barrier to be permeated are used in the formulation. Such penetrants are generally known in the art.

For oral administration, the compounds can be formulated readily by combining the active compounds with pharmaceutically acceptable carriers well known in the art. Such carriers enable the compounds of the invention to be formulated as tablets, pills, capsules, liquids, gels, syrups, slurries, suspensions and the like, for oral ingestion by a patient to be treated. Pharmaceutical preparations for oral use can be obtained with solid excipient, optionally grinding a resulting mixture, and processing the mixture of granules, after adding suitable auxiliaries, if desired, to obtain tablets. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethylcellulose, sodium carboxymethylcellulose, and/or polyvinylpyrrolidone (PVP). If desired, disintegrating agents can be added, such as the cross-linked polyvinyl pyrrolidone, agar, or alginic acid or a salt thereof such as sodium alginate.

Pharmaceutical preparations that can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds can be dissolved or suspended in suitable liquids, such as fatty oils, liquid paraffin, or liquid polyethylene glycols. In addition, stabilizers can be added. All formulations for oral administration should be in dosages suitable for such administration. For buccal administration, the compositions can take the form of tablets or lozenges formulated in conventional manner.

For administration by inhalation, the compounds for use according to the present invention are conveniently delivered in the form of an aerosol spray presentation from pressurized packs or a nebuliser, with the use of a suitable propellant, e.g., dichlorodifluoromethane, trichlorofluoromethane, dichlorotetra-fluoroethane, carbon dioxide or other suitable gas. In the case of a pressurized aerosol the dosage unit can be determined by providing a valve to deliver a metered amount. Capsules and cartridges of e.g., gelatin for use in an inhaler, can be formulated containing a powder mix of the compound and a suitable powder base such as lactose or starch.

The compounds can be formulated for parenteral administration by injection, e.g., by bolus injection or continuous infusion. Formulations for injection can be presented in unit dosage form, e.g., in ampoules or in multidose containers, with an added preservative. The compositions can take such forms as suspensions, solutions or emulsions in oily or aqueous vehicles, and can contain formulatory agents such as suspending, stabilizing and/or dispersing agents.

Pharmaceutical formulations for parenteral administration include aqueous solutions of the active compounds in water-soluble form. Additionally, suspensions of the active compounds can be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions can contain substances that increase the viscosity of the suspension, such as sodium carboxymethyl cellulose, sorbitol, or dextran. Optionally, the suspension can also contain suitable stabilizers or agents that increase the solubility of the compounds to allow for the preparation of highly concentrated solutions. Alternatively, the active ingredient can be in powder form for constitution with a suitable vehicle, e.g., sterile pyrogen-free water, before use. The compounds can also be formulated in rectal compositions such as suppositories or retention enemas, e.g., containing conventional suppository bases such as cocoa butter or other glycerides.

In addition to the formulations described previously, the compounds can also be formulated as a depot preparation. Such long acting formulations can be administered by implantation (for example subcutaneously or intramuscularly) or by intramuscular injection. Thus, for example, the compounds can be formulated with suitable polymeric or hydrophobic materials (for example as an emulsion in an acceptable oil) or ion exchange resins, or as sparingly soluble derivatives, for example, as a sparingly soluble salt.

A pharmaceutical carrier for the hydrophobic compounds of the invention is a cosolvent system comprising benzyl alcohol, a nonpolar surfactant, a water-miscible organic polymer, and an aqueous phase. The cosolvent system can be the VPD co-solvent system. VPD is a solution of 3% w/v benzyl alcohol, 8% w/v of the nonpolar surfactant polysorbate 80, and 65% w/v polyethylene glycol 300, made up to volume in absolute ethanol. The VPD co-solvent system (VPD:5W) consists of VPD diluted 1:1 with a 5% dextrose in water solution. This co-solvent system dissolves hydrophobic compounds well, and itself produces low toxicity upon systemic administration. Naturally, the proportions of a co-solvent system can be varied considerably without destroying its solubility and toxicity characteristics. Furthermore, the identity of the co-solvent components can be varied: for example, other low-toxicity nonpolar surfactants can be used instead of polysorbate 80; the fraction size of polyethylene glycol can be varied; other biocompatible polymers can replace polyethylene glycol, e.g. polyvinyl pyrrolidone; and other sugars or polysaccharides can substitute for dextrose.

Alternatively, other delivery systems for hydrophobic pharmaceutical compounds can be employed. Liposomes and emulsions are well known examples of delivery vehicles or carriers for hydrophobic drugs. Certain organic solvents such as dimethyl sulfoxide also can be employed, although usually at the cost of greater toxicity. Additionally, the compounds can be delivered using a sustained-release system, such as semipermeable matrices of solid hydrophobic polymers containing the therapeutic agent. Various sustained-release materials have been established and are well known by those skilled in the art. Sustained-release capsules can, depending on their chemical nature, release the compounds for a few weeks up to over 100 days. Depending on the chemical nature and the biological stability of the therapeutic reagent, additional strategies for protein and nucleic acid stabilization can be employed.

The pharmaceutical compositions also can comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

The compounds of the invention can be provided as salts with pharmaceutically compatible counterions. Pharmaceutically compatible salts can be formed with many acids, including but not limited to hydrochloric, sulfuric, acetic, lactic, tartaric, malic, succinic, phosphoric, hydrobromic, sulfinic, formic, toluenesulfonic, methanesulfonic, nitric, benzoic, citric, tartaric, maleic, hydroiodic, alkanoic such as acetic, HOOC-(CH₂)ₙ-CH₃ where n is 0-4, and the like. Salts tend to be more soluble in aqueous or other protonic solvents that are the corresponding free base forms. Non-toxic pharmaceutical base addition salts include salts of bases such as sodium, potassium, calcium, ammonium, and the like. Those skilled in the art will recognize a wide variety of non-toxic pharmaceutically acceptable addition salts.

Pharmaceutical compositions of the compounds of the present invention can be formulated and administered through a variety of means, including systemic, localized, or topical administration. Techniques for formulation and administration can be found in "Remington's Pharmaceutical Sciences," Mack Publishing Co., Easton, PA. The mode of administration can be selected to maximize delivery to a desired target site in the body. Suitable routes of administration can, for example, include oral, rectal, transmucosal, transcutaneous, or intestinal administration; parenteral delivery, including intramuscular, subcutaneous, intramedullary injections, as well as intrathecal, direct intraventricular, intravenous, intraperitoneal, intranasal, or intraocular injections.

Alternatively, one can administer the compound in a local rather than systemic manner, for example, via injection of the compound directly into a specific tissue, often in a depot or sustained release formulation.

Pharmaceutical compositions suitable for use in the present invention include compositions wherein the active ingredients are contained in an effective amount to achieve its intended purpose. More specifically, a therapeutically effective amount means an amount effective to prevent development of or to alleviate the existing symptoms of the subject being treated. Determination of the effective amounts is well within the capability of those skilled in the art, especially in light of the detailed disclosure provided herein.

For any compound used in the method of the invention, the therapeutically effective dose can be estimated initially from cell culture assays, as disclosed herein. For example, a dose can be formulated in animal models to achieve a circulating concentration range that includes the EC₅₀ (effective dose for 50% increase) as determined in cell culture, *i.e.,* the concentration of the test compound which achieves a half-maximal inhibition of bacterial cell growth. Such information can be used to more accurately determine useful doses in humans.

It will be understood, however, that the specific dose level for any particular patient will depend upon a variety of factors including the activity of the specific compound employed, the age, body weight, general health, sex, diet, time of administration, route of administration, and rate of excretion, drug combination, the severity of the particular disease undergoing therapy and the judgment of the prescribing physician.

For administration to non-human animals, the drug or a pharmaceutical composition containing the drug may also be added to the animal feed or drinking water. It will be convenient to formulate animal feed and drinking water products with a predetermined dose of the drug so that the animal takes in an appropriate quantity of the drug along with its diet. It will also be convenient to add a premix containing the drug to the feed or drinking water approximately immediately prior to consumption by the animal.

Preferred compounds of the invention will have certain pharmacological properties. Such properties include, but are not limited to oral bioavailability, low toxicity, low serum protein binding and desirable *in vitro* and *in vivo* half-lives. Assays may be used to predict these desirable pharmacological properties. Assays used to predict bioavailability include transport across human intestinal cell monolayers, including Caco-2 cell monolayers. Serum protein binding may be predicted from albumin binding assays. Such assays are described in a review by Oravcová et al. (1996, J. Chromat. B 677: 1-27). Compound half-life is inversely proportional to the frequency of dosage of a compound. *In vitro* half-lives of compounds may be predicted from assays of microsomal half-life as described by Kuhnz and Gieschen (Drug Metabolism and Disposition, (1998) volume 26, pages 1120-1127).

Toxicity and therapeutic efficacy of such compounds can be determined by standard pharmaceutical procedure in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio between LD₅₀ and ED₅₀. Compounds that exhibit high therapeutic indices are preferred. The data obtained from these cell culture assays and animal studies can be used in formulating a range of dosage for use in humans. The dosage of such compounds lies preferably within a range of circulating concentrations that include the ED₅₀ with little or no toxicity. The dosage can vary within this range depending upon the dosage form employed and the route of administration utilized. The exact formulation, route of administration and dosage can be chosen by the individual physician in view of the patient's condition. *(See, e.g.* Fingl et al., 1975, in "The Pharmacological Basis of Therapeutics", Ch.1, p.1).

Dosage amount and interval can be adjusted individually to provide plasma levels of the active moiety that are sufficient to maintain bacterial cell growth inhibitory effects. Usual patient dosages for systemic administration range from 100 - 2000 mg/day. Stated in terms of patient body surface areas, usual dosages range from 50-910 mg/m²/day. Usual average plasma levels should be maintained within 0.1-1000 µM. In cases of local administration or selective uptake, the effective local concentration of the compound cannot be related to plasma concentration.

The compounds of the invention are useful as antibiotics for the treatment of diseases of both animals and humans, including but not limited to actinomycosis, anthrax, bacterial dysentery, botulism, brucellosis, cellulitis, cholera, conjunctivitis, cystitis, diphtheria, bacterial endocarditis, epiglottitis, gastroenteritis, glanders, gonorrhea, Legionnaire's disease, leptospirosis, bacterial meningitis, plague, bacterial pneumonia, puerperal sepsis, rheumatic fever, Rocky Mountain spotted fever, scarlet fever, streptococcal pharyngitis, syphilis, tetanus, tuberculosis, tularemia, typhoid fever, typhus, and pertussis.

The disclosures in this application of all articles and references, including patents, are incorporated herein by reference.

The compounds of the invention comprise a novei class of broad-spectrum antibiotics. Medically-important bacterial species that provide appropriate targets for the antibacterial activity of the inhibitors of the invention includes gram-positive bacteria, including cocci such as *Staphylococcus* species and *Streptococcus* species; acid-fast bacterium, including *Mycobacterium* species; bacilli, including *Bacillus* species, *Corynebacterium* species and *Clostridium* species; filamentous bacteria, including *Actinomyces* species and *Streptomyces* species; gram-negative bacteria, including cocci such as *Neisseria* species and *Acinetobacter* species; bacilli, such as *Pseudomonas* species, *Brucella* species, *Agrobacterium* species, *Bordetella* species, *Escherichia* species, *Shigella* species, *Yersinia* species, *Salmonella* species, *Klebsiella* species, *Enterobacter* species, *Haemophilus* species, *Pasteurella* species, and *Streptobacillus* species; spirochetal species, *Campylobacter* species, *Vibrio* species; and intracellular bacteria including *Rickettsiae* species and *Chlamydia* species.

Specific bacterial species that are targets for the antibiotics of the invention include *Staphylococcus aureus; Staphylococcus epidermidis, Staphylococcus saprophyticus; Streptococcus pyogenes; Streptococcus agalactiae; Streptococcus pneumoniae; Enterococcus faecalis; Enterococcus faecium; Bacillus anthracis; Mycobacterium avium, Mycobacterium tuberculosis, Acinetobacter baumannii; Corynebacterium diphtheria; Clostridium perfringens; Clostridium botulinum; Clostridium tetani*; *Neisseria gonorrhoeae; Neisseria meningitidis; Pseudomonas aeruginosa*; *Legionella pneumophila*; *Escherichia coli*; *Yersinia pestis*; *Haemophilus influenzae*; *Helicobacter pylori*; *Campylobacter fetus; Campylobacter jejuni, Vibrio cholerae*; *Vibrio parahemolyticus*; *Trepomena pallidum*; *Actinomyces israelii*; *Rickettsia prowazekii*; *Rickettsia rickettsii*; *Chlamydia trachomatis*; *Chlamydia psittaci*; *Brucella abortus*; *Agrobacterium tumefaciens*; *and Francisella tularensis.*

In carrying out the procedures of the present invention it is of course to be understood that reference to particular buffers, media, reagents, cells, culture conditions and the like are not intended to be limiting, but are to be read so as to include all related materials that one of ordinary skill in the art would recognize as being of interest or value in the particular context in which that discussion is presented. For example, it is often possible to substitute one buffer system or culture medium for another and still achieve similar, if not identical, results. Those of skill in the art will have sufficient knowledge of such systems and methodologies so as to be able, without undue experimentation, to make such substitutions as will optimally serve their purposes in using the methods and procedures disclosed herein.

The invention is described in more detail in the following non-limiting examples. It is to be understood that these methods and examples in no way limit the invention to the embodiments described herein and that other embodiments and uses will no doubt suggest themselves to those skilled in the art.

The compounds of this invention are evaluated for their antibacterial activity as per the guidelines and procedures prescribed by the National Committee for Clinical Laboratory Standards (NCCLS) (cf., NCCLS Document M7-A3, 1993 -Antimicrobial Susceptibility Testing).

### Protocol for MIC Determination

A useful protocol for MIC determination is as follows:
1. Approximately 2.5 mg of the compounds to be tested was weighed into cryovials.
2. 5 mg/ml stock solutions were made by adding DMSO to the samples accordingly.
3. 256 µg/ml working solutions were made by using the 5 mg/ml stock solutions and adding sterile distilled water accordingly.
4. A Beckman 2000 Automated Workstation was programmed to load 96 well plates with broth and compounds as follows:
   - 100 µl of the appropriate broth was added to columns 1-11
   - 200 µl of the appropriate broth was added to column 12
   - 100 µl of compounds at the 256 µg/ml working solution were added to column 1 (one compound per row)
   - Two-fold serial dilutions were done from column 1 to 10
   - Column 11 served as the growth control
5. The 10 organism panel was plated from stock vials stored at -80°C and incubated for 24 hours at 34°C. The organisms were then sub-cultured and incubated for 24 hours at 34°C.
   - The inoculums were first prepared in sterile distilled water with a target of 0.09-0.11 absorbance at 620 nm wavelength
   - A 1/100 dilution was made into the appropriate broth
   - 100 µl of broth with organism was added to columns 1-11
   - Column 12 served as the blank control
6. The completed 96 well plates were incubated for 24 hours at 34°C. The 96 well plates were then read using a Beckman Automated Plate Reader at 650 nm wavelength. The MIC was determined through calculations involving the growth control (column 11) and blank control (column 12).

### BORINATE COMPLEXES

This procedure was used to obtain the results in the following tables. Representative microbiological data for the compounds 10 to 123 are shown in Tables 1 to 4 as MIC (Minimum Inhibitory Concentration) with the values expressed as micrograms per ml.

Thus, the invention provides antibiotics that are generically called borinic acid complexes, most preferably derived from disubstituted borinic acids.

The synthesis of the compounds of the invention is accomplished in several formats. Reaction scheme #1 demonstrate the synthesis of the intermediate borinic acids, and their subsequent conversion to the desired borinic acid complexes. When R* and R** are identical, the reaction of two equivalents of an arylmagnesium halide (or aryllithium) with trialkyl borate, followed by acidic hydrolysis affords the desired borinic acid **5**. When R* and R** are not identical, the reaction of an equivalent of an arylmagnesium halide (or aryllithium) with appropriate aryl(dialkoxy)borane (**4**), heteroaryl(dialkoxy)borane or alkyl(dialkoxy)borane (alkoxy group comprised of methoxy, ethoxy, isopropoxy, or propoxy moiety), followed by acidic hydrolysis affords the unsymmetrical borinic acids **6** in excellent yields. Where applicable, the reaction of the alkylene esters (**3**, T = nothing, CH₂, CMe₂) with the appropriate organolithium or organomagnesium reactant is convenient.

As shown in Scheme 1, the borinic acid complexes are obtained from the precursor borinic acids by reaction with 1 equivalent of the desired heterocyclic ligand in suitable solvents (i.e., ethanol, isopropanol, dioxane, ether, toluene, dimethylformamide, N-methylpyrrolidone, or tetrahydrofuran).

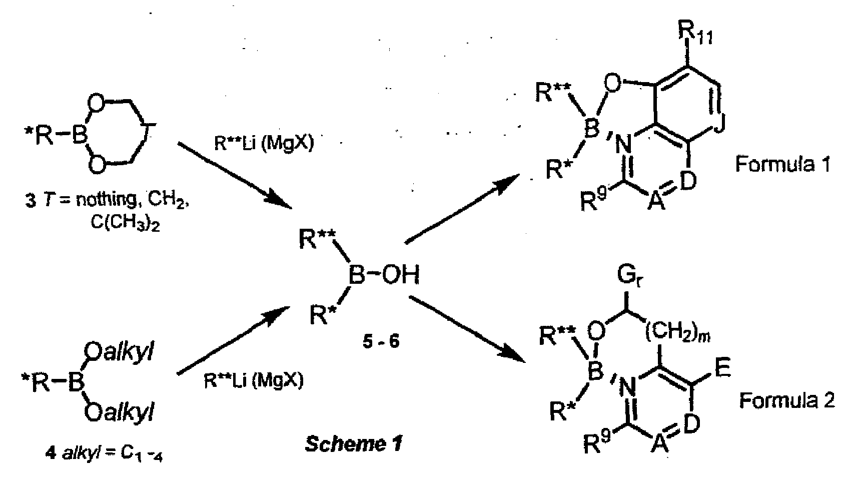

In certain situations, compounds of the invention may contain one or more asymmetric carbon atoms, so that the compounds can exist in different stereoisomeric forms. These compounds can be, for example, racemates or optically active forms. In these situations, the single enantiomers, *i*.*e*., optically active forms, can be obtained by asymmetric synthesis or by resolution of the racemates. Resolution of the racemates can be accomplishes, for example, by conventional methods such as crystallization in the presence of a resolving agent, or chromatography, using, for example a chiral HPLC column.

Representative compounds of the present invention include, but are not limited to the compounds disclosed herein and their pharmaceutically acceptable acid and base addition salts. In addition, if the compound of the invention is obtained as an acid addition salt, the free base can be obtained by basifying a solution of the acid salt. Conversely, if the product is a free base, an addition salt, particularly a pharmaceutically acceptable addition salt, may be produced by dissolving the free base in a suitable organic solvent and treating the solution with an acid, in accordance with conventional procedures for preparing acid addition salts from base compounds. In a preferred embodiment, the compounds of the invention comprise any of compounds 10 -123 (Tables 1, 2, 3 and 4), and variants thereof.

| **Table 1. Antibacterial Profile Against Select Gram-positive and Gram-negative Pathogens** | | | | | | | | | |
|---|---|---|---|---|---|---|---|---|---|
| **Cmp** | **R*** | **R**** | **Ligand** | ***S. aureus* ATCC 29213** | ***S. epidermidis* ATCC 12228** | ***S. pneumoniae* ATCC 6301** | ***E. faecalis* ATCC 29212** | ***E. faecium* CT-26** | ***M. catarrhalis* ATCC 25238** |
| **10** | 3-ClC₈H₄ | 3-ClC₈H₄ | 8-hydroxyquinoline | 1 | 2 | 2 | 32 | 4 | NA |
| **11** | 4-Me-3-ClC₈H₃ | 4-Me-3-ClC₈H₃ | 4-hydroxybenzimidazole | 0.125 | 4 | NA | 8 | 8 | NA |
| **12** | 3-ClC₈H₄ | 3-ClC₈H₄ | 5-fluoro-8-hydroxyquinoline | 0.125 | 2 | 2 | 8 | 4 | 2 |
| **13** | 3-ClC₈H₄ | 3-ClC₈H₄ | 5-chloro-8-hydroxyquinoline | 0.125 | 1 | 1 | 64 | 2 | 0.25 |
| **14** | 3-ClC₈H₄ | 3-ClC₈H₄ | 4-methyl-8-hydroxyquinoline | 0.125 | 1 | 1 | 64 | 4 | 0.5 |
| **16** | 2-F4-ClC₈H₃ | 3-FC₈H₄ | 8-hydroxyquinoline | 0.125 | 1 | 2 | 16 | 4 | 0.5 |
| **16** | 4-Nle-3-ClC₈H₃ | 4-Me-3-ClC₈H₃ | 2-HO₂C-4-hydroxy-5,7-dichloroquinoline | 0.25 | 0.5 | NA | 0.5 | 0.25 | NA |
| **17** | 3-ClC₈H₄ | 3-ClC₈H₄ | 2-amino-8-hydroxyquinoline | 0.25 | 2 | 2 | 8 | 8 | 2 |
| **18** | 3-ClC₈H₄ | 3-Cl-4-FC₈H₃ | 8-hydroxyquinoline | 0.25 | 1 | 2 | 8 | 4 | 1 |
| **19** | 3-ClC₈H₄ | 3-ClC₈H₄ | 5-cyano-8-hydroxyquinoline | 0.25 | 2 | 4 | 16 | 4 | 0.5 |
| **20** | 3-ClC₈H₄ | 3-Cl-5-FC₈H₃ | 8-hydroxyquinoline | 0.25 | 1 | 2 | 8 | 4 | 2 |
| **21** | 3-ClC₈H₄ | 3-FC₈H₄ | 5-cyano-8-hydroxyquinoline | 0.5 | 4 | 2 | 16 | 8 | 0.25 |
| **22** | 3-ClC₈H₄ | 3-FC₈H₄ | 5-nitro-8-hydroxyquinoline | 0.5 | 4 | 2 | 64 | 16 | 0.12 |
| **23** | 3-ClC₈H₄ | 3-FC₈H₄ | 5-chloro-7-chloro-8-hydroxyquinoline | 0.5 | 16 | 8 | 64 | 16 | 0.12 |
| **24** | 3-ClC₈H₄ | 3-FC₈H₄ | 5-bromo-7-bromo-8-hydroxyquinoline | 0.5 | 8 | 8 | 64 | 32 | 0.12 |
| **28** | 3-ClC₈H₄ | 3-ClC₈H₄ | 2-carboxy-4-hydroxy-8-methoxyquinoline | 0.5 | 8 | 2 | 16 | 16 | 2 |
| **26** | 2-thienyl | Me | 8-hydroxyquinoline | 0.5 | 1 | NA | 4 | 4 | NA |
| **27** | 3-NCC₈H₄ | 4-Me-3-ClC₈H₃ | 8-hydroxyquinoline | 0.5 | 1 | 1 | 8 | 2 | 1 |
| **28** | 3,4-Cl₂C₈H₃ | 3-FC₈H₄ | 8-hydroxyquinoline | 0.5 | 1 | 2 | 4 | 2 | 1 |
| **29** | 2,4-Cl₂C₈H₃ | 3-FC₈H₄ | 8-hydroxyquinoline | 0.5 | 1 | 2 | 8 | 2 | 0.5 |
| **30** | 3,4-Cl₂C₈H₃ | 3,4-Cl₂C₈H₃ | 8-hydroxyquinoline | 1 | 0.5 | NA | 2 | 2 | NA |
| **31** | 4-Nle-3-ClC₈H₃ | 4-Me-3-ClC₈H₃ | 2-carboxy-4-hydroxyquinoline | 1 | 1 | NA | 2 | 1 | NA |
| **32** | 3-ClC₈H₄ | 3-FC₈H₄ | 8-hydroxyquinoline | 1 | 1 | 1 | 16 | 2 | 2 |
| **33** | 3-Cl-5-FC₈H₃ | 3-MeC₈H₄ | 8-hydroxyquinoline | 1 | 1 | 1 | 8 | 2 | 2 |
| **34** | 3-ClC₈H₄ | 3-FC₈H₄ | 5-fluoro-8-hydroxyquinoline | 1 | 2 | 2 | 8 | 4 | 1 |
| **35** | 3-ClC₈H₄ | 3-MeSC₈H₄ | 5-fluoro-8-hydroxyquinoline | 1 | 2 | 2 | 8 | 4 | 2 |
| **36** | 3-ClC₆H₄ | 2-thienyl | 8-hydroxyquinoline | 1 | 1 | 2 | 8 | 2 | 4 |
| **37** | 3-Me-4-ClC₆H₃ | 3-NCC₆H₄ | 8-hydroxyquinoline | 1 | 1 | 1 | 8 | 2 | 1 |
| **38** | 2-FC₆H₄ | 3-NCC₆H₄ | 8-hydroxyquinoline | 1 | 1 | 2 | 16 | 2 | 1 |
| **39** | 3-ClC₆H₄ | 3-NCC₆H₄ | 8-hydroxyquinoline | 1 | 1 | 1 | 8 | 2 | 2 |
| **40** | 3-NCC₆H₄ | Vinyl | 8-hydroxyquinoline | 1 | 1 | 1 | 8 | 2 | 2 |
| **41** | 3-NCC₈H₄ | Ethynyl | 8-hydroxyquinoline | 1 | 1 | 1 | 4 | 2 | 1 |
| **42** | 3-FC₈H₄ | Ethynyl | 8-hydroxyquinoline | 1 | 1 | 1 | 8 | 2 | 1 |
| **43** | 2-ClC₈H₄ | Ethynyl | 8-hydroxyquinoline | 1 | 1 | 1 | 8 | 2 | 1 |
| **44** | Ethynyl | Ethynyl | 8-hydroxyquinoline | 1 | 1 | 1 | 16 | 2 | 0.25 |
| **46** | 3,5-F₂C₈H₃ | Ethynyl | 8-hydroxyquinoline | 1 | 1 | 1 | 8 | 1 | 1 |
| **46** | 3,5-Cl₂C₈H₃ | Ethynyl | 8-hydroxyquinoline | 1 | 1 | 1 | 4 | 1 | 1 |
| **47** | 3,4-Cl₂C₈H₃ | Ethynyl | 8-hydroxyquinoline | 1 | 1 | 1 | 8 | 2 | 1 |
| **48** | 3-Cl-4-FC₈H₃ | Ethynyl | 6-hydroxyquinoline | 1 | 1 | 1 | 8 | 2 | 2 |
| **49** | 4-ClC₈H₄ | 4-ClC₈H₄ | 5-chloro-8-hydroxyquinoline | 2 | 1 | 1 | 16 | 2 | 0.25 |
| **50** | 4-ClC₈H₄ | 4-ClC₈H₄ | 8-hydroxyquinoline | 2 | 2 | 2 | 4 | 4 | NA |
| **51** | 3-FC₈H₄ | 3-FC₈H₄ | 8-hydroxyquinoline | 2 | 1 | NA | 8 | 2 | NA |
| **52** | -Me-3-ClC₈H₃ | 4-Me-3-ClC₈H₃ | 5-hydroxyquinoline | 2 | 4 | NA | 8 | 16 | NA |
| **53** | -NCC₆H₄ | 3-NCC₆H₄ | 8-hydroxyquinoline | 2 | 2 | NA | 64 | 4 | 2 |
| **54** | 4-ClC₈H₄ | 4-Cl-3-FC₈H₃ | 8-hydroxyquinoline | 2 | 1 | 2 | 8 | 4 | 1 |
| **55** | 4-Cl-3-FC₈H₃ | 4-Cl-3-FC₈H₃ | 8-hydroxyquinoline | 2 | 1 | 4 | 4 | 2 | 4 |
| **56** | 3-MeC₈H₄ | 3,5-Cl₂C₈H₃ | 8-hydroxyquinoline | 2 | 2 | 2 | 8 | 4 | 4 |
| **57** | 4-ClC₈H₄ | 4-FC₆H₄ | 5-fluoro-8-hydroxyquinoline | 2 | 2 | 4 | 16 | 4 | 1 |
| **58** | 3-ClC₈H₄ | 4-FC₈H₄ | 5-fluoro-8-hydroxyquinoline | 2 | 2 | 2 | 8 | 4 | 0.5 |
| **59** | 3-ClC₈H₄ | 4-MeSC₈H₄ | 8-hydroxyquinoline | 2 | 1 | 1 | 8 | 4 | 2 |
| **60** | 4-ClC₈H₄ | 3-MeSC₈H₄ | 8-hydroxyquinoline | 2 | 1 | 2 | 8 | 4 | 2 |
| **61** | 3-ClC₈H₄ | cyclopropyl | 9-hydroxyquinoline | 2 | 1 | 1 | 16 | 2 | 2 |
| **62** | 4-ClC₈H₄ | 3-MeSC₈H₄ | 5-fluoro-8-hydroxyquinoline | 2 | 2 | 2 | 6 | 4 | 2 |
| **63** | 4-ClC₈H₄ | 4-MeSC₈H₄ | 8-hydroxyquinoline | 2 | 1 | 2 | 8 | 4 | 1 |
| **64** | 4-ClC₈H₄ | 4-MeSC₈H₄ | 5-fluoro-8-hydroxyquinoline | 2 | 2 | 4 | 8 | 8 | 1 |
| **65** | 4-ClC₈H₄ | 4-Cl-3-HOC₈H₃ | 8-hydroxyquinoline | 2 | 2 | 2 | 16 | 4 | 4 |
| **66** | 4-ClC₈H₄ | 3-FC₈H₄ | 4-methyl-8-hydroxyquinoline | 2 | 1 | 1 | 64 | 4 | 0.5 |
| **67** | 3-ClC₈H₄ | 3-(DMISO)C₈H₄ | 4-methyl-8-hydroxyquinoline | 2 | 1 | 1 | 64 | 4 | 0.5 |
| **66** | 3-FC₈H₄ | 3-(DMISO)C₈H₄ | 8-hydroxyquinoline | 2 | 2 | 16 | 32 | 4 | 0.12 |
| **69** | 3-(DMISO)C₈H₄ | cyclopropyl | 8-hydroxyquinoline | 2 | 1 | 2 | 64 | 4 | 1 |
| **70** | 3-FC₈H₄ | cyclopropyl | 6-hydroxyquinoline | 2 | 1 | 1 | 64 | 2 | 0.5 |
| **71** | 3-FC₈H₄ | 4-NCC₈H₃ | 5-chloro-7-chloro-8-hydroxyquinoline | 2 | 2 | 8 | 64 | 4 | 0.12 |
| **72** | 3-(DMISO)C₈H₄ | 3-(DMISO)C₈H₄ | 8-hydroxyquinoline | 4 | 2 | 4 | 64 | 4 | NA |
| **73** | 3-(DMISO)C₈H₄ | Vinyl | 8-hydroxyquinoline | 2 | 1 | 2 | 64 | 8 | 0.25 |
| **74** | 4-FC₈H₄ | 4-NCC₈H₄ | 8-hydroxyquinoline | 2 | 1 | 2 | 32 | 2 | 1 |
| **75** | 3-ClC₈H₄ | 3-MeSC₈H₄ | 8-hydroxyquinoline | 2 | 1 | 2 | 64 | 4 | NA |
| **76** | 4-Me-3-ClC₈H₃ | 2-thienyl | 8-hydroxyquinoline | 2 | 1 | NA | 8 | 4 | NA |
| **77** | 3-ClC₈H₄ | 2-MeC₈H₄ | 8-hydroxyquinoline | 2 | 1 | 1 | 8 | 4 | 2 |
| **78** | 3-ClC₈H₄ | 2-MeOC₈H₄ | 8-hydroxyquinoline | 2 | 1 | 1 | 8 | 2 | 2 |
| **79** | 3-ClC₈H₄ | 2-Ms-4-ClC₈H₃ | 8-hydroxyquinoline | 2 | 2 | 2 | 8 | 4 | 2 |
| **80** | 4-Cl-3-MeC₈H₃ | 4-Cl-3-MeC₈H₃ | 8-hydroxyquinoline | 2 | 1 | 2 | 4 | 4 | 2 |
| **81** | 3-ClC₈H₄ | 3-Cl-6-MeOC₈H₃ | 8-hydroxyquinoline | 2 | 1 | 2 | 8 | 4 | 2 |
| **82** | 3,5-Cl₂C₈H₃ | 4-(Me₂NC₂H₄)OC₆H₄ | 8-hydroxyquinoline | 2 | 2 | 2 | 8 | 2 | 4 |
| **83** | 4-BrC₈H₄ | 4-(Me₂NC₂H₄)OC₈H₄ | 8-hydroxyquinoline | 2 | 1 | 2 | 4 | 4 | 2 |
| **84** | 3-ClC₈H₄ | 4-F-3-MeC₈H₃ | 8-hydroxyquinoline | 2 | 1 | 2 | 8 | 4 | 4 |
| **85** | 3-Me-4-ClC₈H₃ | 3-F-4-ClC₈H₃ | 8-hydroxyquinoline | 2 | 1 | 2 | 4 | 4 | 2 |
| **86** | 3-FC₈H₄ | 4-Cl-3-MeC₈H₃ | 8-hydroxyquinoline | 2 | 1 | 2 | 8 | 4 | 2 |
| **87** | 3-FC₈H₄ | 3-F-4-ClC₈H₃ | 8-hydroxyquinoline | 2 | 1 | 2 | 8 | 4 | 1 |
| **88** | 3-Cl-6-FC₈H₃ | 3-NCC₈H₄ | 8-hydroxyquinoline | 2 | 2 | 2 | 8 | 2 | 2 |
| **89** | 2,5-F₂C₈H₃ | 3-NCC₈H₄ | 8-hydroxyquinoline | 2 | 1 | 1 | 8 | 2 | 2 |
| **90** | 4-F-3-ClC₈H₃ | 3-NCC₈H₄ | 8-hydroxyquinoline | 2 | 2 | 1 | 8 | 2 | 2 |
| **91** | 3-Me-4-ClC₈H₃ | 4-NCC₈H₄ | 8-hydroxyquinoline | 2 | 1 | 2 | 8 | 2 | 1 |
| **92** | 2,5-F₂C₈H₃ | 4-NCC₈H₄ | 6-hydroxyquinoline | 2 | 1 | 2 | 8 | 4 | 1 |
| **93** | 3-Cl-6-FC₈H₃ | 4-NCC₈H₄ | 8-hydroxyquinoline | 2 | 1 | 1 | 8 | 4 | 1 |
| **94** | 3-Cl-6-MeOC₈H₃ | 4-NCC₈H₄ | 8-hydroxyguinoline | 2 | 1 | 1 | 8 | 4 | 2 |
| **95** | 4-NCC₈H₄ | Ethynyl | 8-hydroxyquinoline | 2 | 1 | 2 | 8 | 2 | 1 |
| **96** | 4-ClC₈H₄ | 3,4-F₂C₈H₃ | 8-hydroxyquinoline | 2 | 1 | 2 | 8 | 2 | 1 |
| **97** | 4-ClC₈H₄ | 4-Me-3-FC₈H₃ | 8-hydroxyquinoline | 2 | 1 | 1 | 8 | 2 | 1 |
| **98** | 4-ClC₈H₄ | 3,5-F₂C₈H₃ | 8-hydroxyquinoline | 2 | 1 | 1 | 8 | 4 | 1 |
| **98** | 3-CF₃-4-ClC₈H₃ | 3-FC₈H₄ | 8-hydroxyquinoline | 2 | 1 | 2 | 8 | 2 | 1 |
| **100** | 4-ClC₈H₄ | 3-F-5-CF₃C₈H₃ | 8-hydroxyquinoline | 2 | 1 | 2 | 4 | 2 | 1 |
| *Ciprofloxacin* | | | | 0.125 | 0.125 | 0.5 | 0.5 | 64 | 0.125 |
| *Cloxacillin* | | | | 0.125 | 0.25 | 0.125 | 16 | 64 | 1 |
| *Imipenem* | | | | 0.125 | 0.125 | 0.125 | 1 | 64 | 0.125 |
| *Ceftriaxone* | | | | 2 | 1 | 0.125 | 64 | 64 | 0.125 |
| *Meropenem* | | | | 0.06 | 0.06 | | 2 | | |
| *Erythromycin* | | | | 0.5 | 0.5 | | 2 | | |
| *Pen G* | | | | 0.5 | 16 | 0.125 | 1 | 32 | 0.125 |
| DMISO = 4,4-dimethyloxazolin-2-yl | | | | | | | | | |

| **Table 2. Antibacterial Profile Against Select Gram-positive and Gram-negative Pathogens** | | | | | | | | | |
|---|---|---|---|---|---|---|---|---|---|
| **Cmp** | **R*** | **R**** | **Ligand** | ***S. aureus* ATCC 29213** | ***S. epidermidis* ATCC 12228** | **S. *pneumonia* ATCC 6301** | ***E. faecalis* ATCC 29212** | ***E. faecium* CT-28** | ***H. influenzae* ATCC 49766** |
| **101** | 3-ClC₈H₄ | 3-ClC₈H₄ | 1-(2-morpholino-4-yl-ethyl)-imidazoleacetate | 0.12 | 4 | 16 | 64 | 64 | 4 |
| **102** | 3-ClC₈H₄ | 3-ClC₈H₄ | 2-hydroxyisopropyl-3-hydroxypyridine | 0.5 | 1 | 0.25 | 64 | 2 | 64 |
| **103** | 4-ClC₈H₄ | 4-ClC₈H₄ | 2-hydroxyisopropyl-3-hydroxypyridine | 0.25 | 0.5 | 0.5 | 4 | 1 | 64 |
| **104** | 4-Me-3-ClC₈H₃ | 4-Me-3-ClC₈H₃ | 2-hydroxymethyl-1N-benzylimidazole | 0.5 | 4 | NA | 16 | 32 | 8 |
| **105** | 3-ClC₈H₄ | 3-ClC₈H₄ | 2-hydroxymethylpyridine | 0.125 | 4 | 4 | 32 | 32 | 4 |
| **106** | 4-Me-3-ClC₈H₃ | 4-Me-3-ClC₈H₃ | 2-pyridylacetic acid | 0.5 | 4 | NA | 64 | 64 | 64 |
| **107** | 4-Me-3-ClC₈H₃ | 4-Me-3-ClC₈H₃ | 3-(2-hydroxyethoxy)picolinic acid | 0.125 | 4 | NA | 16 | 8 | 32 |
| **108** | 4-Me-3-ClC₈CH₃ | 4-Me-3-ClC₈H₃ | 3-(N-morpholinylethoxy)picolinic acid | 0.25 | 4 | NA | 4 | 2 | 64 |
| **109** | 4-Me-3-ClC₈H₃ | 4-Me-3-ClC₈H₃ | 3-(OCH₂CH₂CH₂O₂H)picolinic acid | 1 | 4 | 4 | 32 | 16 | 16 |
| **110** | 4-Me-3-ClC₈H₃ | 4-Me-3-ClC₈H₃ | 3-carboxypicolinic acid | 0.125 | 4 | NA | 8 | 8 | 8 |
| **111** | 4-Me-3-ClC₈H₃ | 4-Me-3-ClC₈H₃ | 3-hydroxypicolinic acid | 2 | 1 | NA | 2 | 2 | 64 |
| **112** | 4-Me-3-ClC₈H₃ | 4-CH₃C₈H₄ | 3-hydroxypicolinic acid | 4 | 2 | NA | 4 | 8 | 64 |
| **113** | 4-Me-3-ClC₈H₃ | Phenylethyl | 3-hydroxypicolinic acid | 0.5 | 1 | NA | 2 | 64 | 64 |
| **114** | 3-ClC₈H₄ | 3-ClC₈H₄ | 3-hydroxypicolinic acid | 0.125 | 8 | NA | 64 | 64 | 16 |
| **115** | 4-EtO-3-ClC₈H₃ | 4-EtO-ClC₈H₃ | 3-hydroxypicolinic acid | 2 | 2 | 1 | 8 | 16 | 64 |
| **116** | 2-Cl-5-Br-6-FC₈H₂ | 2-F-4-ClC₈H₃ | 3-hydroxypicolinic acid | 2 | 1 | 0.25 | 4 | 4 | 64 |
| **117** | 2-Me-4-ClC₈H₃ | 3-ClC₈H₄ | 3-hydroxypicolinic acid | 2 | 1 | 0.5 | 4 | 4 | 16 |
| **118** | 2-Me-4-ClC₈H₃ | 2-Me-4-ClC₈H₃ | 3-hydroxypicolinic acid | 1 | 0.25 | 0.12 | 1 | 1 | 16 |
| **119** | 4-Me-3-ClC₈H₃ | 4-Me-3-ClC₈H₃ | 3-OAc-picolinic acid | 2 | 1 | NA | 2 | 2 | 64 |
| **120** | 4-Me-3-ClC₈H₃ | 4-Me-3-ClC₈H₃ | 4-hydroxybenzimidazole | 0.125 | 4 | NA | 8 | 8 | |
| **121** | 3-ClC₈H₄ | 3-ClC₈H₄ | 4-hydroxyethylimidazole | 0.125 | 4 | 8 | 32 | 32 | 4 |
| **122** | 3-ClC₈H₄ | 3-ClC₈H₄ | 6-amino-3-hydroxypicolinic acid | 0.25 | 4 | 16 | 32 | 32 | 8 |
| **123** | 3-ClC₈H₄ | 3-ClC₈H₄ | Imidazole acetic acid | 0.125 | 2 | 8 | 32 | 32 | 8 |
| *Ceftriaxone* | | | | 2 | 1 | <0.125 | 64 | 64 | 0.12 |
| *Ciprofloxacin* | | | | 0.12 | 0.12 | 0.5 | 0.5 | 64 | 0.12 |
| *Cloxacillin* | | | | 0.12 | 0.25 | 0.12 | 16 | 64 | 8 |
| *Erythromycin* | | | | 0.5 | 0.5 | NA | 2 | NA | 4 |
| *Imipenem* | | | | 0.12 | 0.12 | <0.125 | 1 | 64 | 2 |
| *Meropenem* | | | | 0.06 | 0.06 | NA | 2 | NA | 0.06 |
| *Pen G* | | | | 0.5 | 16 | <0.125 | 1 | 32 | 0.12 |

| **Table 3. Anti-mycobacterium In vitro Activity** | | | |
|---|---|---|---|
| | ***M. tuberculosis* MIC (mcg/mL)** | | |
| **Compound** | **H37Rv*** | **P2SP1**** | **P1SP2**** |
| **10** | 0.387 | 0.387 | 0.387 |
| **50** | 0.387 | 0.387 | 0.387 |
| **51** | 0.387 | 0.387 | 0.387 |
| **53** | 0.775 | 0.775 | 0.387 |
| **55** | 0.775 | 0.775 | 0.387 |
| **65** | 0.775 | 0.775 | 0.775 |
| **72** | 0.775 | 0.775 | 0.775 |
| **75** | 0.775 | 0.775 | 0.775 |
| *Isoniazid (INH)* | <0.062 | >8 | >8 |
| *Rifampicin* | <0.125 | 16 | >16 |
| *Ethambutol* | <1 | 8 | 8 |
| *Ethionamide* | 1 | >64 | 32 |
| *p-aminosalicylate* | <0.25 | 32 | 16 |
| *Of*/*oxacin* | 4 | 32 | 16 |
| *Streptomycin* | <2 | <2 | <2 |
| *Kanamycin* | <2 | <2 | <2 |
| *cycloserine* | 8 | 8 | 8 |

| | | | |
|---|---|---|---|
| *Sensitive strain **Multi-drug resistant strain | | | |

| **Table 4.** | | ***C. albicans* ATCC 90028** |
|---|---|---|
| | **Compound** | |
| **Antifungal Activity for Select Borinic Acid Complexes** | **10** | 2 |
| | **50** | 2 |
| | **51** | 1 |
| | **52** | 2 |
| | **53** | 1 |
| | **55** | 1 |
| | **65** | 0.5 |
| | **72** | 4 |
| | **76** | 2 |

The present invention also encompasses the acylated prodrugs of the compounds of the invention. Those skilled in the art will recognize various synthetic methodologies which may be employed to prepare non-toxic pharmaceutically acceptable addition salts and acylated prodrugs of the inventive compounds.

Tables 1, 2 and 3 also contain inhibitory activity for known antibiotics, shown at the end of each of the tables.

### EXAMPLES

Proton NMR are recorded on Varian AS 400 spectrometer and chemical shifts are reported as δ (ppm) down field from tetramethylsilane. Mass spectra are determined on Micromass Quattro II. Example numbers refer to compounds.

### Formation of ethylene glycol boronate ester (Compound 3, T= nothing)

### General procedure

Boronic acid was dissolved in dry THF or dry diethyl ether (~10 mL/g) under nitrogen. Ethylene glycol (1 molar equivalent) was added to the reaction and the reaction was heated to reflux for 1 to 4 hours. Reaction was cooled to room temperature and solvent was removed under reduced pressure leaving the ethylene glycol ester as an oil or a solid. In cases where an oil was obtained or a solid that dissolved in hexane, dry hexane was added and removed under reduced pressure. The product was then placed under high vacuum for several hours. In cases where a solid was obtained that did not dissolve in hexane, the solid was collected by filtration and washed with cold hexane.

### 3-Cyanophenylboronic acid ethylene glycol ester (3a)

3-Cyanophenyl boronic acid (1 g, 6.8 mmol) was dissolved in dry THF (10 mL) under nitrogen. Ethylene glycol (379 µL, 422 mg, 6.8 mmol) was added and the reaction was heated to reflux for 4 hours then cooled to room temperature. THF was removed by rotary evaporator to give a white solid. Cold hexane was added and the product was collected by filtration giving a white solid (1.18 g, quant. yield). ¹H-NMR (300.058 MHz, DMSO-d6) δ ppm 7.92-8.01 (3H, m), 7.50-7.64 (1H, m), 4.35 (4H, s)

### Thiophene 3-boronic acid ethylene glycol ester (3b)

Thiophen-3-boronic acid (1 g, 7.8 mmol) was dissolved in dry THF (10 mL) under nitrogen. Ethylene glycol (435 µL, 484 mg, 7.8 mmol) was added and the reaction was heated to reflux for 1 hour then cooled to room temperature. THF was removed by rotary evaporator to give a white solid. Hexane was added, dissolving the solid and removed by rotary evaporation. The product was placed under high vacuum to yield a tan solid (1.17g, 97%). ¹H-NMR (300.058 MHz, CDCl₃) δ ppm 7.93. (1H, s), 7.3-7.4 (2H, m), 4.35 (4H, s).

### Formation of unsymmetrical borinic acid (6) from boronic acid ethylene glycol ester

### General procedure A: Grignard methodology

Boronic acid ethylene glycol ester was dissolved in dry THF (10-20 mUg) under nitrogen. Solution was cooled to -78 °C in an acetone/dry ice bath or to 0 °C in an ice/water bath. Grignard reagent (0.95 to 1.2 molar equivalent) was added dropwise to the cooled solution. The reaction was warmed to room temperature and stirred for 3-18 hours. 6N HCl (2 mUg) was added and solvent was removed under reduced vacuum. Product was extracted into diethyl ether (40 mL/g) and washed with water (3 x equal volume). Organic layer was dried (MgSO₄), filtered and the solvent was removed by rotary evaporation giving the crude product, which is either purified by column chromatography or taken onto the next step without purification. Alternative work-up: if the borinic acid product contained a basic group such as an amine or pyridine, then after stirring at room temperature for 3 - 18 hours water (2 mL/g) was added and the pH adjusted to 5-7. Product was extracted into diethyl ether (40 mL/g) and washed with water (3 x equal volume). Organic layer was dried (MgSO₄), filtered and the solvent was removed by rotary evaporation giving the crude product, which is either purified by column chromatography or taken onto the next step without purification.

### (4-Cyanophenyl)(3-fluorophenyl)borinic acid (6a)

4-Cyanophenyl boronic acid ethylene glycol ester (500 mg, 2.89 mmol) was dissolved in dry THF under nitrogen. The solution was cooled to -78°C in an acetone/dry ice bath and 3-fluorophenylmagnesium bromide (1M in THF)(2.74 mL, 2.74 mmol, 0.95 molar equivalent) was added dropwise to the cold solution. The reaction was allowed to warm slowly to room temperature and stirred for 18 hours. 6N HCl (1 mL) was added to the reaction causing a cloudy appearance and the solvent was removed using a rotary evaporator. The product was extracted into diethyl ether (20 mL) and washed with water (3 x 20 mL). The organic layer was dried (MgSO4), filtered and the solvent removed using a rotary evaporator to yield the crude product as an oily solid. This was taken onto the next step without purification.

### General procedure B: (Hetero)aryl-lithium methodology

The (hetero)aryl-bromide or iodide was dissolved in dry THF (20-30 mUg) under nitrogen and degassed. The solution was cooled to -78 °C in an acetone/dry ice bath and n-, sec- or tert-butyllithium in THF or other solvent (1.5-2.4 molar equivalents) was added to the cooled solution dropwise generally causing the solution to turn deep yellow. The boronic acid ethylene glycol ester (1 molar equivalent) was dissolved in dry THF or diethyl ether (2-5 mL/g) under nitrogen. The boronic acid ethylene glycol ester in THF was added dropwise to the cooled aryl-lithium solution generally causing the solution to turn pale yellow. The reaction was warmed to room temperature and stirred for 3-18 hours. 6N HCl (2-4 mL/g) was added and solvent was removed under reduced vacuum. Product was extracted into diethyl ether (40 mL/g) and washed with water (3 x equal volume). Organic layer was dried (MgSO₄), filtered and the solvent was removed by rotary evaporation giving the crude product, which is either purified by column chromatography or taken onto the next step without purification. Alternative work-up: if the borinic acid product contained a basic group such as an amine or pyridine then after stirring at room temperature for 3 - 18 hours water (2 mUg) was added and the pH adjusted to 5-7. Product was extracted into diethyl ether (40 mUg) and washed with water (3 x equal volume). Organic layer was dried (MgSO₄), filtered and the solvent was removed by rotary evaporation giving the crude product, which is either purified by column chromatography or taken onto the next step without purification.

### (3-Thiophene)(3-chlorophenyl)borinic acid (6b)

3-Chloro-bromobenzene (447 µL, 728 mg, 3.8 mmol) was dissolved in dry THF (15 mL) under nitrogen. The solution was degassed and cooled to -78 °C in an acetone/dry ice bath. tert-Butyllithium (1.7M in THF)(4.47 mL, 7.6 mmol, 2 molar equivalent) was added to the cooled solution dropwise causing the solution to turn deep yellow. The solution was stirred at -78 °C while 3-thiopheneboronic acid ethylene glycol ester (586 mg) was dissolved in dry diethyl ether (1 mL). The boronic ester solution was then added dropwise to the cooled solution causing the colour to change to pale yellow. The reaction was warmed to room temperature and stirred for 18 hours. 6N HCl (2 mL) was added and the reaction was stirred for 1 hour. The solvent was removed using a rotary evaporator. The product was extracted into diethyl ether (10 mL) and washed with water (2 x 10 mL). The organic layer was dried (MgS04), filtered and the solvent removed using a rotary evaporator to yield the crude product as an orange oil. The product was purified by column chromatography using silica gel and hexane:ethyl acetate 5:1 as eluent giving the pure product as a clear oil (614 mg, 73%).

### (3-Chlorophenyl)vinylbornic acid (6c)

This was prepared by a similar process as described for 6b by the reaction of 3-cyanophenyl boronic acid ethylene glycol ester with vinyllithium.

### (3-Fluoro-5-chlorophenyl)ethynylborinic acid (6d)

This was prepared by a similar process as described for 6b by the reaction of 3-fluoro-5-chlorophenyl boronic acid ethylene glycol ester with ethynyllithium.

### (4-Methyl-3-chlorophenyl)(2-thienyl)borinic acid (6e)

This was prepared by a similar process as described for 6b by the reaction of 2-thienylboronic acid ethylene glycol ester with 4-methyl-3-chlorophenyllithium..

### (4-Cyanophenyl)ethynylborinic acid (6f)

This was prepared by a similar process as described for 6b by the reaction of 4-cyanophenylboronic acid ethylene glycol ester with ethynyllithium.

### (3-Fluorophenyl)cyclopropylborinic acid (6g)

This was prepared by a similar process as described for 6b by the reaction of 3-fluorophenylboronic acid ethylene glycol ester with cyclopropyllithium.

### (3-Thienyl)methylborinic acid (6h)

This was prepared by a similar process as described for 6b by the reaction of 3-thienylboronic acid ethylene glycol ester with methyllithium.

### (4-Pyridyl)phenylborinic acid (6i)

This was prepared by a similar process as described for 6b by the reaction of phenylboronic acid ethylene glycol ester with 4-pyridyllithium.

### (3-Cyanophenyl)(2-fluorophenyl)borinic acid (6j)

This was prepared by a similar process as described for 6b by the reaction of 3-cyanophenylboronic acid ethylene glycol ester with 2-fluorophenyllithium.

### Formation of symmetrical borinic acid (5) by reaction of organometallics with trialkyl borates. Bis(4-chlorophenyl)borinic acid (5a) Procedure C)

A cold solution (-78 °C) of trimethyl borate (0.37 ml) in dry tetrahydrofuran (THF, 25 ml) was treated dropwise with 4-chlorophenylmagnesium bromide (6.75 ml, 1M solution in ether). The reaction mixture was stirred at -78°C for 1 h and then stirred for 18 h at room temperature. The solvent was removed under reduced pressure. The resultant residue was stirred with 100 ml of ether and 15 ml of 6N hydrochloric acid. Organic layer was separated and aqueous layer was extracted with ether (2 X 100 ml). The combined organic extract was washed with brine and dried over anhydrous magnesium sulfate. Solvent was removed to give light yellowish solid. The product was chromatographed over silica gel·(Hex: Ether =1:1) to give 420 mg of borinic acid. ¹H NMR (400 MHz, CDCl₃) δ: 5.84 (s. OH), 7.46 (d, 4H, Ar-H), 7.72 (d, 4H, Ar-H).

### Bis(3-Chloro-4-methylphenyl)borinic acid (5b)

In a similar manner as for 5a, the titled compound was obtained from the reaction of 3-chloro-4-methylphenylmagnesium bromide with trimethyl borate. The product was obtained by chromatography over silica gel.

### Bis(3-Fluoro-4-methylphenyl)borinic acid (5c)

In a similar manner as for 5a, the titled compound was obtained from the reaction of 3-fluoro-4-methylphenyllithium with trimethyl borate. The product was obtained by chromatography over silica gel.

### Bis(3-Chloro-4-methoxyphenyl)borinic acid (5d)

In a similar manner as for 5a, the titled compound was obtained from the reaction of 3-chloro-4-methoxyphenyllithium with trimethyl borate. The product was obtained by chromatography over silica gel.

### Bis(3-Fluoro-4-methoxyphenyl)borinic acid 5e

In a similar manner as for 5a, the titled compound was obtained from the reaction of 3-fluoro-4-methoxyphenyllithium with trimethyl borate. The product was obtained by chromatography over silica gel.

### Formation of unsymmetrical borinic acids (6) by reaction of organometallics with alkyl(aryl)dialkoxyboranes. (4-Chlorophenyl)methyl-borinic acid (6k) (Procedure D)

To 4-chlorophenylmagnesium bromide (5.5. ml; 1M solution in ether) at -78°C, di(isopropoxy)methylborane (1 ml, 0.78 g) was added dropwise via syringe. The reaction mixture was stirred at -78°C for 1h and then stirred overnight at ambient temperature. The reaction mixture was treated dropwise with 100 ml of ether and 15ml of 6N hydrochloric acid, and stirred for 1 h. Organic layer was separated and aqueous layer was extracted with ether (2 X 100 ml). The combined organic extract was washed with brine and dried over anhydrous sodium sulfate. Solvent was removed under reduce pressure to give 1.1 g of oil. ¹H NMR of the product was consistent for (4-chlorophenyl)methyl) borinic acid.

### (4-Fluorophenyl)methylborinic acid (6m

In a similar manner as for 6k, the titled compound was obtained from the reaction of 4-fluorophenylmagnesium bromide with di(isopropoxy)methylborane. The product was obtained by chromatography over silica gel.

### (4-Biphenyl)methylborinic acid (6n)

In a similar manner as for 6k, the titled compound was obtained from the reaction of 4-biphenyllithium with di(isopropoxy)methylborane. The product was obtained by chromatography over silica gel.

### (3-Chloro-4-methylphenyl)methylborinic acid (6o)

In a similar manner as for 6k, the titled compound was obtained from the reaction of 3-chloro-4-methylphenyllithium with di(isopropoxy)methylborane. The product was obtained by chromatography over silica gel.

### (3-Chloro-4-methoxyphenyl)methylborinic acid (6p)

In a similar manner as for 6k, the titled compound was obtained from the reaction of 3-chloro-4-methoxyphenyllithium with di(isopropoxy)methylborane. The product was obtained by chromatography over silica gel.

### (4-Dimethylaminophenyl)methylborinic acid (6q)

In a similar manner as for 6k, the titled compound was obtained from the reaction of 4-dimethylaminophenyllithium with di(isopropoxy)methylborane. The product was obtained by chromatography over silica gel.

### (3-Chloro4-dimethylaminophenyl)vinylborinic acid (6r)

In a similar manner as for 6k, the titled compound was obtained from the reaction of 3-chloro-4-dimethylaminophenyllithium with di(butoxyvinyl)-borane. The product was obtained by chromatography over silica gel.

### Bis(3-Chlorophenyl)borinic acid 4-(hydroxyeethyl)imidazole ester (121)

To a solution of bis(3-chlorophenyl)borinic acid (0.4 g, 1.428 mmol) in ethanol (10 ml), 4-(hydroxyethyl)imidazole hydrochloride (0.191 g, 1.428 mmol), sodium bicarbonate ( 0.180 g, 2.143 mmol) were added and the reaction mixture was stirred at room temperature for 18 h. Salt was removed by filtration. Filtrate was concentrated and treated with hexane to afford the product as a solid and was collected by filtration. (450 mg, 84.9% yield)

### Bis(4-Chlorophenyl)borinic acid 4-(hydroxymethyl)imidazole ester (126)

In a similar manner as in Example 121, the titled compound was obtained from the reaction of bis(4-chlorophenyl)borinic acid with 4-(hydroxymethyl)imidazole hydrochloride. The product was obtained as white crystals.

### Bis(3-Chloro-4-methylphenyl)birinic acid 1-benzyl-4-(hydroxymethyl)-imidazole ester (127)

To a solution of 1-benzyl-4-(hydroxymethyl)imidazole (96 mg, 0.521 mmol) in methanol (5 ml), bis(3-chloro-4-methylphenyl)borinic, acid (121 mg, 0.521 mmol) was added and the reaction mixture was stirred at room temperature for 2 h. Solvent was removed under reduced pressure and the residue was treated with hexane to give a solid. The product was isolated by filtration and washed with hexane to give product (193 mg, 83%). ¹H NMR (CDCl₃) δ: 2.3 (s, 6H, 2XCH₃), 4.8 (brs, 2H, CH₂), 5.1 (brs, 2H, CH₂), 6.9-7.4 (complex, 13H, Ar-H); MS (ES⁺)(m/z) 448.78, MF C₂₅H₂₃BCl₂N₂O.

### Bis3-Chloro-4-methylphenyl)borinic acid 1-methyl-2-(hydroxymethyl)-imidazole ester (128)

In a similar manner as in Example 127, the titled compound was obtained from the reaction of bis(3-chloro-4-methylphenyl)borinic acid with 1-methyl-2-(hydroxy-methyl)imidazole hydrochloride. The product was obtained as white crystals.

### Bis(3-Chloro-4-methylphenyl)borinic acid 1-ethyl-2-(hydroxymethyl)-imidazole ester (129)

In a similar manner as in Example 127, the titled compound was obtained from the reaction of bis(3-chloro-4-methylphenyl)borinic acid with 1-ethyl-2-(hydroxy-methyl)imidazole hydrochloride. The product was obtained as white crystals.

### Bis(3-Chloro-4-methylphenyl)borinic acid 1-methyl-4-(hydroxymethyl)-imidazole ester (130)

In a similar manner as in Example 127, the titled compound was obtained from the reaction of bis(3-chloro-4-methylphenyl)borinic acid with 1-methyl-4-(hydroxy-methyl)imidazole hydrochloride. The product was obtained as white crystals.

### Bis(3-Chloro-4-methytphenyl)borinic acid 2-pyridylethanol (131

In a similar manner as in Example 121, the titled compound was obtained from the reaction of bis(3-chloro-4-methylphenyl)borinic acid with 2-pyridylethanol. The product was obtained as white crystals.

### Bis(4-Chlorophenyl)borinic acid 2-pyridytmethanol (132)

In a similar manner as in Example 121, the titled compound was obtained from the reaction of bis(4-chlorophenyl)borinic acid with 2-pyridylmethanol. The product was obtained as white crystals.

### Bis(4-Fluorophenyl)borinic acid 2-pyridylmethanol (133)

In a similar manner as in Example 121, the titled compound was obtained from the reaction of bis(4-fluorophenyl)borinic acid with 2-pyridylmethanol. The product was obtained as white crystals.

### HYDROXYQUINOLINE DERIVATIVES

### Bis(3-Chlorophenyl)borinic acid 8-hdroxyquinoline ester (10)

A solution of bis(3-chlorophenyl)borinic acid (0.18 g) in ethanol (1 ml) and 8-hydroxyquinoline (0.105 g) was stirred at 5 °C. The reaction mixture was then stirred at ambient temperature, and a yellow solid precipitate formed. The reaction mixture was stirred for additional four hours. The product was isolated by filtration, washed with hexane and air dried to give 160 mg of complex.

### Bis(3-Chlorophenyl)bonnic acid 5-Fluoro-8-hydroxyquinoline ester (12)

In a similar manner as in Example 10, the titled compound was obtained from the reaction of bis(3-chlorophenyl)borinic acid with 5-fluoro-8-hydroxyquinoline. The product was obtained as yellow crystals.

### Bis(3-Chloropheny)borinic acid 5-chloro-8-hydroxyquinoline ester (13)

In a similar manner as in Example 10 the titled compound was obtained from the reaction of bis(3-chlorophenyl)borinic acid with 5-chloro-8-hydroxyquinoline. The product was obtained as yellow crystals.

### Bis(3-Chlorophenyl)borinic acid 5-cyano-8-hydroxyquinoline ester (19)

In a similar manner as in Example 10, the titled compound was obtained from the reaction of bis(3-chlorophenyl)borinic acid with 5-cyano-8-hydroxyquinoline. The product was obtained as yellow crystals.

### (2-Thienyl)methylborinic acid 8-hydroxyquinoline ester (26)

In a similar manner as in Example 10, the titled compound was obtained from the reaction of (2-thienyl)methylbonic acid with 8-hydroxyquinoline. The product was obtained as yellow crystals.

### (3-Chlorophenyl)(2-thienyl)borinic acid 8-hydroxyquinoline ester (36

In a similar manner as in Example 10, the titled compound was obtained from the reaction of (3-chlorophenyl)(2-thienyl)borinic acid with 8-hydroxyquinoline. The product was obtained as yellow crystals.

### (3-Cyanophenyl)vinylborinic acid 8-hydroxyquinoline ester (40)

In a similar manner as in Example 10, the titled compound was obtained from the reaction of (3-cyanophenyl)vinylborinic acid with 8-hydroxyquinoline. The product was obtained as yellow crystals.

### (2-Chlorophenyl)ethynylborinic acid 8-Hydroxyquinoline ester (43)

In a similar manner as in Example 10, the titled compound was obtained from the reaction of (2-chlorophenyl)ethynylborinic acid with 8-hydroxyquinoline. The product was obtained as yellow crystals.

### Bis(ethynyl)borinic acid 8-Hydroxyquinoline (44) (XXI)

In a similar manner as in Example 10, the titled compound was obtained from the reaction of bis(ethynyl)borinic acid with 8-hydroxyquinoline. The product was obtained as light yellow crystals.

### (3-Fluorophenyl)cyclopropylborinic acid 8-hydroxyquinoline ester (70)

In a similar manner as in Example 10, the titled compound was obtained from the reaction of (3-fluorophenyl)cyclopropylborinic acid with 8-hydroxyquinoline. The product was obtained as light yellow crystals.

In a preferred embodiment, the present invention includes the compounds specifically recited herein, and pharmaceutically acceptable salts thereof, and compositions of any of these compounds where these comprise a pharmaceutically acceptable carrier. Most preferred are compounds having the structure of any of the compounds listed in Tables 1, 2, 3 or 4, especially those having the structure of compound 10 to 108, compound 111-112, or compound 116-120. In such compounds, the ligand is as described elsewhere herein, where the ligand is attached to the boron through the indicated reactive groups.

The present invention also relates to a method for treating a microbial-caused disease in a patient afflicted therewith and/or preventing such infection in a patient at risk of becoming so-infected, comprising administering to said patient a therapeutically effective amount of any of the compounds of the invention, preferably one or more of those listed in Tables 1 to 4. In one aspect, the compounds of the invention have anti-bacterial (i.e., bactericidal) and anti-fungal (i.e., fungicidal) activity.

In a preferred embodiment, the microbe is a bacterium, preferably a gram positive bacterium, wherein said gram positive bacterium is a member selected from the group consisting of *Staphylococcus* species, *Streptococcus* species, *Bacillus* species, *Mycobacterium* species, *Corynebacterium* species, *Clostridium* species, *Actinomyces* species, *Enterococcus* species, and *Streptomyces* species.

In another preferred embodiment of such method, the bacterium is a gram negative bacterium, preferably one selected from the group consisting of *Acinetobacter* species, Neisseria species, *Pseudomonas* species, *Brucella* species, *Agrobacterium* species, *Bordetella* species, *Escherichia* species, *Shigella* species, *Yersinia* species, *Salmonella* species, *Klebsiella* species, *Enterobacter* species, *Haemophilus* species, *Pasteurella* species, *Streptobacillus* species, spirochetal species, *Campylobacter* species, *Vibrio* species, and *Helicobacter* species.

In a highly preferred embodiment of the present invention, the bacterium is a member selected from the group consisting of *Staphylococcus aureus; Staphylococcus epidermidis; Staphylococcus saprophyticus; Streptococcus pyogenes; Streptococcus agalactiae; Streptococcus pneumonia; Enterococcus faecalis; Enterococcus faecium; Bacillus anthracis; Mycobacterium avium; Mycobacterium tuberculosis; Acinetobacter baumanii; Corynebacterium diphtheria; Clostridium perfringens; Clostridium botulinum; Clostridium tetani; Neisseria gonorrhoeae; Neisseria meningitidis; Pseudomonas aeruginosa; Legionella pneumophila; Escherichia coli; Yersinia pestis; Haemophilus influenzae; Helicobacter pylori; Campylobacter fetus; Campylobacter jejuni; Vibrio cholerae; Vibrio parahemolyticus; Trepomena pallidum; Actinomyces israelii; Rickettsia prowazekii; Rickettsia rickettsii; Chlamydia trachomatis; Chlamydia psittaci; Brucella abortus; Agrobacterium tumefaciens;* and *Francisella tularensis.*

## Claims

1. A compound which is a member selected from:
a chemical compound having a structure of the following Formula: 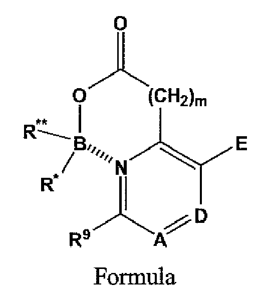
wherein B is boron, O is oxygen
wherein R* and R** are each independently selected from substituted or unsubstituted alkyl (C₁ - C₄), substituted or unsubstituted cycloalkyl (C₃ - C₆), substituted or unsubstituted vinyl, substituted or unsubstituted alkynyl, substituted or unsubstituted benzyl, substituted or unsubstituted phenyl, and substituted or unsubstituted heterocycle,
and wherein A is CH, CR¹⁰ or N,
and wherein D is N, CH, or CR¹²
and wherein E is H, OH, alkoxy or 2-(morpholino)ethoxy,
and wherein m = 0-2,
wherein R¹² is selected from (CH₂)ₖOH (where k = 1, 2 or 3), CH₂NH₂, CH₂NH-alkyl, CH₂N(alkyl)₂, CO₂H, CO₂alkyl, CONH₂, OH, alkoxy, aryloxy, SH, S-alkyl, S-aryl, SO₂alkyl, SO₃H, SCF₃, CN, halogen, CF₃, NO₂, NH₂, 2*-amino, 3*-amino, NH₂SO₂ and CONH₂,
and wherein R⁹ and R¹⁰ are members independently selected from the group consisting of hydrogen, alkyl, (CH₂)ₙOH (n = 1 to 3), CH₂NH₂, CH₂NHalkyl, CH₂N(alkyl)₂, halogen, CHO, CH=NOH, CO₂H, CO₂-alkyl, S-alkyl, SO₂-alkyl, S-aryl, NH₂, alkoxy, CF₃, SCF₃, NO₂, SO₃H and OH,
and salts of said chemical compound having a structure of said Formula.

2. A compound according to claims 1 wherein in the Formula R* is a substituted or unsubstituted alkyl (C₁ - C₄), and /or R** is a substituted or unsubstituted alkyl (C₁ - C₄), and preferably R* and R** are the same substituted or unsubstituted alkyl (C₁ - C₄).

3. A compound according to claim 1 wherein in the Formula R* is a substituted or unsubstituted cycloalkyl (C₃ - C₆), and /or R** is a substituted or unsubstituted cycloalkyl (C₃ - C₆), and preferably R* and R** are the same substituted or unsubstituted cycloalkyl (C₃ - C₆).

4. A compound according to claim 1 wherein in the Formula R* is a substituted or unsubstituted vinyl, and /or R** is a substituted or unsubstituted vinyl, and preferably R* and R** are the same substituted or unsubstituted vinyl.

5. A compound according to claim 1 wherein in the Formula R* is a substituted or unsubstituted alkynyl, and /or R** is a substituted or unsubstituted alkynyl, and preferably R* and R** are the same substituted or unsubstituted alkynyl.

6. A compound according to claim 1
wherein in the Formula R* and R** are each a substituted or unsubstituted cycloalkyl (C₃ - C₆), and wherein E is N-(morpholinyl)ethoxy or alkoxy greater than C_{4.}

7. A compound according to claim 1 wherein in the Formula R* and R** are each a substituted or unsubstituted cycloalkyl (C₃ - C₆),
and wherein A is N,
and wherein D is N.

8. A compound according to claim 1
wherein in the Formula R* and R** are each 3-chloro-4-methylphenyl, R⁹ is H, A is CH, D is CH, E is OH, and m = 0.

9. A compound according to claim 1
wherein in the Formula R* and R** are each 2-methyl-4-chlorophenyl, R⁹ is H, A is CH, D is CH, E is OH, and m = 0.

10. A compound according to claim 1 wherein in the Formula R* is a substituted or unsubstituted benzyl, and /or R** is a substituted or unsubstituted benzyl, and preferably R* and R** are the same substituted or unsubstituted benzyl.

11. A compound according to claim 1 wherein in the Formula R* is a substituted or unsubstituted heterocycle, and /or R** is a substituted or unsubstituted heterocycle, and preferably R* and R** are the same substituted or unsubstituted heterocycle.

12. A compound according to claim 1 wherein in the Formula A is CH, and wherein D is CH,
and wherein E is OH,
and wherein m = 0,
and wherein R⁹ is H.

13. A solvent adduct having the structure of the following Formula: 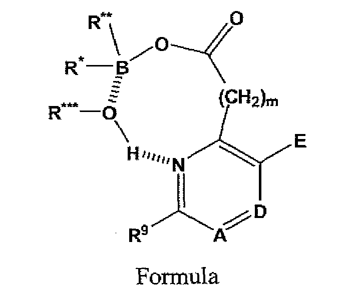 wherein B is boron, O is oxygen
wherein R* and R** are each independently selected from substituted or unsubstituted alkyl (C₁ - C₄), substituted or unsubstituted cycloalkyl (C₃-C₆), substituted or unsubstituted vinyl, substituted or unsubstituted alkynyl, substituted or unsubstituted benzyl, substituted or unsubstituted phenyl, and substituted or unsubstituted heterocycle,
and wherein A is CH, CR¹⁰ or N,
and wherein D is N, CH, or CR¹²,
and wherein E is OH, alkoxy or 2-(morpholino)ethoxy,
and wherein m = 0-2,
wherein R¹² is selected from (CH₂)ₖOH (where k = 1, 2 or 3), CH₂NH₂, CH₂NH-alkyl, CH₂N(alkyl)₂, CO₂H, CO₂,alkyl, CONH₂, OH, alkoxy, aryloxy, SH, S-alkyl, S-aryl, SO₂alkyl, SO₃H, SCF₃, CN, halogen, CF₃, NO₂, NH₂, 2*-amino, 3*-amino, NH₂SO₂ and CONH₂,
and wherein R⁹ and R¹⁰ are members independently selected from the group consisting of hydrogen, alkyl, (CH₂)ₙOH (n = 1 to 3), CH₂NH₂, CH₂NHalkyl, CH₂N(alkyl)₂, halogen, CHO, CH=NOH, CO₂H, CO₂-alkyl, S-alkyl, SO₂-alkyl, S-aryl, NH₂, alkoxy, CF₃, SCF₃, NO₂, SO₃H and OH,
and wherein R*** is H or alkyl
and salts of said chemical compound having a structure of said Formula.

14. The compound of claim 4, wherein said vinyl has the structure: 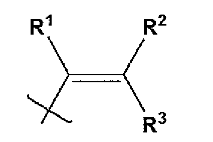 wherein R¹, R², and R³ are each independently selected from the group consisting of hydrogen, alkyl, aryl, substituted aryl, benzyl, substituted benzyl, (CH₂)ₖOH (where k = 1, 2 or 3), CH₂NH₂, CH₂NH-alkyl, CH₂N(alkyl)₂, CO₂H, CO₂alkyl, CONH₂, S-alkyl, S-aryl, SO₂alkyl, SO₃H, SCF₃, CN, halogen, CF₃ and NO₂.

15. The compound of claim 5, wherein said alkynyl has the structure: 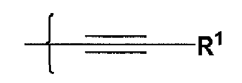 wherein R¹ is selected from the group consisting of hydrogen, alkyl, aryl, substituted aryl, benzyl, substituted benzyl, (CH₂)ₖOH (where k = 1, 2 or 3), CH₂NH₂, CH₂NH-alkyl, CH₂N(alkyl)₂, CO₂H, CO₂alkyl, CONH₂, S-alkyl, S-aryl, SO₂alkyl, SO₃H, SCF₃, CN, halogen, CF₃ and NO₂.

16. A compound according to claim 1 wherein in the Formula R* is a substituted or unsubstituted phenyl, and /or R** is a substituted or unsubstituted phenyl, and preferably R* and R** are the same substituted or unsubstituted phenyl.

17. A compound according to claim 1 wherein R* and R** are the same and are members selected from substituted or unsubstituted alkyl (C₁ - C₄), substituted or unsubstituted cycloalkyl (C₃ - C₆), substituted or unsubstituted vinyl, substituted or unsubstituted alkynyl, substituted or unsubstituted benzyl, substituted or unsubstituted phenyl, and substituted or unsubstituted heterocycle,
and wherein R* and R** are other than a phenyl or substituted phenyl.

18. A compound according to claim 1 wherein in the Formula R* and R** are the same and are members selected from substituted or unsubstituted alkyl (C₁ C₄), substituted or unsubstituted cycloalkyl (C₃ - C₆), substituted or unsubstituted vinyl, substituted or unsubstituted alkynyl, substituted or unsubstituted benzyl, substituted or unsubstituted phenyl, and substituted or unsubstituted heterocycle,
and wherein E is OH,
and wherein m = 0.

19. A compound according to claim 1 wherein in the Formula R* and R** are the same and are members selected from substituted or unsubstituted alkyl (C₁ - C₄), substituted or unsubstituted cycloalkyl (C₃-C₆), substituted or unsubstituted vinyl, substituted or unsubstituted alkynyl, substituted or unsubstituted benzyl, substituted or unsubstituted phenyl, and substituted or unsubstituted heterocycle,
and wherein D is CR¹²,
and wherein R¹² is selected from (CH₂)ₖOH (where k = 1, 2 or 3), CH₂NH₂, CH₂NH-alkyl, CH₂N(alkyl)₂, CO₂H, CO₂alkyl, CONH₂, OH, alkoxy (greater than C₄), aryloxy, SH, S-alkyl, S-aryl, SO₂alkyl, SO₃H, SCF₃, CN, NO₂, NH₂SO₂ and CONH₂.

20. A compound according to claim 1 wherein in the Formula R* and R** are the same and are members selected from substituted or unsubstituted alkyl (C₁ - C₄), substituted or unsubstituted cycloalkyl (C₃ - C6), substituted or unsubstituted vinyl, substituted or unsubstituted alkynyl, substituted or unsubstituted benzyl, substituted or unsubstituted phenyl, and substituted or unsubstituted heterocycle,
and wherein E is N-(morpholinyl)ethoxy or alkoxy greater than C₄.

21. A compound according to claim 1 wherein in the Formula R* and R** are the same and are members selected from substituted or unsubstituted alkyl (C₁- C₄), substituted or unsubstituted cycloalkyl (C₃ - C₆), substituted or unsubstituted vinyl, substituted or unsubstituted alkynyl, substituted or unsubstituted benzyl, substituted or unsubstituted phenyl, and substituted or unsubstituted heterocycle,
wherein A or D is nitrogen.

22. A compound according to claim 1 wherein in the Formula R* and R** are the same and are members selected from substituted or unsubstituted alkyl (C₁ - C₄), substituted or unsubstituted cycloalkyl (C₃ - C₆), substituted or unsubstituted vinyl, substituted or unsubstituted alkynyl, substituted or unsubstituted benzyl, substituted or unsubstituted phenyl, and substituted or unsubstituted heterocycle,
and wherein E is H, OH, alkoxy or 2-(morpholino)ethoxy,
and wherein m = 2.

23. A compound according to claim 1 wherein in the Formula R* and R** are the same and are members selected from substituted phenyl substituted with 1 to 5 substituents each of which is independently selected from alkyl (greater than C₆), aryl, substituted aryl, benzyl, substituted benzyl, (CH₂)ₖOH (where k = 1,2 or 3), CH₂NH₂, CH₂NH-alkyl, CH₂N(alkyl)₂, CO₂H, CO₂alkyl, CONH₂, CONHalkyl, CON(alkyl)₂, OH, alkoxy (greater than C₆), aryloxy, SH, S-alkyl, S-azyl, SO₂alkyl, SO₃H, SCF₃, CN, NO₂, NH₂, 2°-amino, 3°-amino, NH₂SO₂, OCH₂CH₂NH₂, OCH₂CH₂NHalkyl, OCH₂CH₂N(alkyl)₂, oxazolid in-2-yl and alkyl substituted oxazolidin-2-yl.

24. The compound of claim 16 wherein said phenyl has the structure 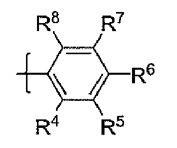 wherein R⁴, R⁵, R⁶, R⁷ and R⁸ are each independently selected from the group consisting of hydrogen, alkyl, aryl, substituted aryl, benzyl, substituted benzyl, (CH₂)ₖOH (where k = 1, 2 or 3), CH₂NH₂, CH₂NH-alkyl, CH₂N(alkyl)₂, CO₂H, CO₂alkyl, CONH₂, CONHalkyl, CON(alkyl)₂, OH, alkoxy, aryloxy, SH, S-alkyl, S-aryl, SO₂alkyl, SO₃H, SCF₃, CN, halogen, CF₃, NO₂, NH₂, 2°-amino, 3°-amino, NH₂SO₂, OCH₂CH₂NH₂, OCH₂CH₂NHalkyl, OCH₂CH₂N(alkyl)₂, oxazolidin-2-yl, or alkyl substituted oxazolidin-2-yl.

25. The compound of claim 10 wherein said benzyl has the structure 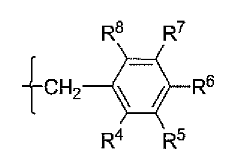 wherein R⁴, R⁵, R⁶, R⁷ and R⁸ are each independently selected from the group consisting of alkyl, aryl, substituted aryl, benzyl, substituted benzyl, (CH₂)ₖOH (where k = 1, 2 or 3), CH₂NH₂, CH₂NH-alkyl, CH₂N(alkyl)₂, CO₂H, CO₂alkyl, CONH₂, CONHalkyl, CON(alkyl)₂, OH, alkoxy, aryloxy, SH, S-alkyl, S-aryl, SO₂alkyl, SO₃H, SCF₃, CN, halogen, CF₃, NO₂, NH₂, 2°-amino, 3°-amino, NH₂SO₂, OCH₂CH₂NH₂, OCH₂CH₂N-Halkyl, OCH₂CH₂N(alkyl)₂, oxazolidin-2-yl, and alkyl substituted oxazolid in-2-yl.

26. The compound of claim 11 wherein said heterocycle has the structure 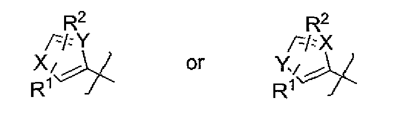 wherein X= CH=CH, N=CH, NR¹³ (wherein R¹³ = H, alkyl, aryl or benzyl), O, or S
and wherein Y = CH or N
and wherein R¹, R², and R³ are each independently selected from the group consisting of hydrogen, alkyl, aryl, substituted aryl, benzyl, substituted benzyl, (CH₂)ₖOH (where k = 1, 2 or 3), CH₂NH₂, CH₂NH-akyl, CH₂N(alkyl)₂, CO₂H, CO₂alkyl, CONH₂, S-alkyl, S-aryl, SO₂alkyl, SO₃H, SCF₃, CN, halogen, CF₃, NO₂.

27. A compound according to claim 1 wherein in the Formula R* and R** are the same and are members selected from substituted or unsubstituted alkyl (C₁ - C₄), substituted or unsubstituted cycloalkyl (C₃ - C₆), substituted or unsubstituted vinyl, substituted or unsubstituted alkynyl, substituted or unsubstituted benzyl, substituted or unsubstituted phenyl, and substituted or unsubstituted heterocycle,
and wherein A is CH,
and wherein D is CH,
and wherein E is OH,
and wherein m = 0,
and wherein R⁹ is H.

28. A compound having the structure which is a member selected from: 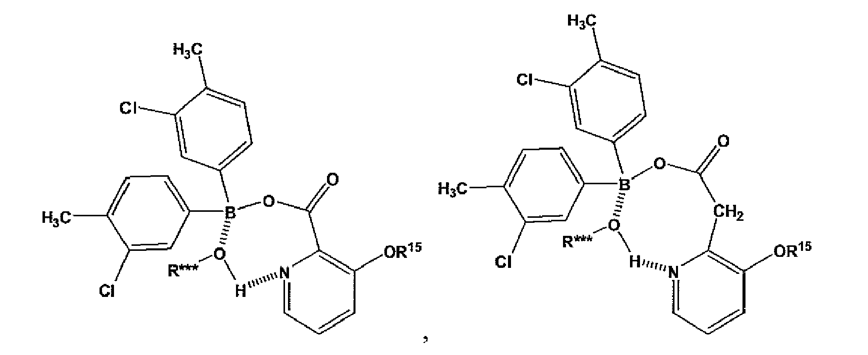 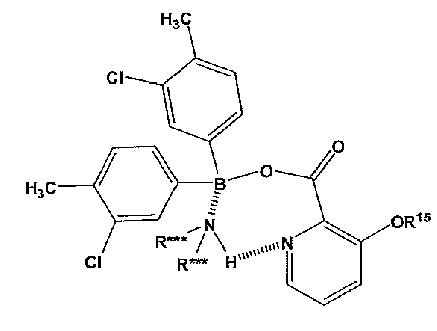 and 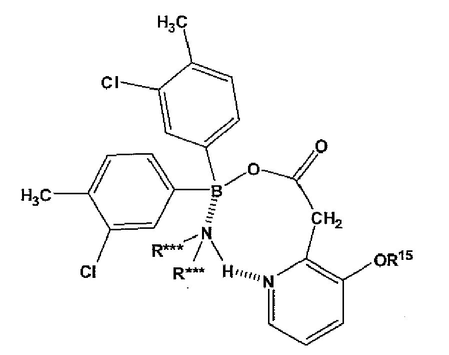 in which
each R*** is a member independently selected from H and alkyl; and
R¹⁵ is a member selected from H and a pharmaceutically compatible counterion.

29. A compound according to claim 1 wherein in the Formula R* and R** are members selected from substituted or unsubstituted phenyl, with the proviso that R* and R** are not both unsubstituted phenyl.

30. A compound according to claim 1 wherein in the Formula R* and R** are the same and are members selected from substituted or unsubstituted alkyl (C₁ - C₄), substituted or unsubstituted cycloalkyl (C₃ - C6), substituted or unsubstituted vinyl, substituted or unsubstituted alkynyl, substituted or unsubstituted benzyl, substituted phenyl, and substituted or unsubstituted heterocycle,
and wherein R⁹ is a member selected from the group consisting of hydrogen, alkyl, (CH₂)ₙOH (n = 1 to 3), CH₂NH₂, CH₂NHalkyl, CH₂N(alkyl)₂, halogen, CHO, CH=NOH, CO₂H, CO₂-alkyl, S-alkyl, SO₂-alkyl, S-aryl, NH₂, alkoxy, CF₃, SCF₃, NO₂, SO₃H and OH,
R¹⁰ is selected from alkyl (greater than C₄), (CH₂)ₙOH (n = 1, 2 or 3), CH₂NH₂, CH₂NHalkyl, CH₂N(alkyl)₂, CHO, CH=NOH, CO₂H, CO₂-alkyl, S-alkyl, SO₂-alkyl, S-aryl, alkoxy (greater than C₄), SCF₃ and NO₂.

31. The compound of claim 30 wherein said phenyl has the structure 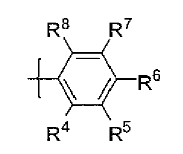 wherein R⁴, R⁵, R⁶, R⁷ and R⁸ are each independently selected from the group consisting of hydrogen, alkyl, aryl, substituted aryl, benzyl, substituted benzyl, (CH₂)ₖOH (where k = 1,2 or 3), CH₂NH₂, CH₂NH-alkyl, CH₂N(alkyl)₂, CO₂H, CO₂alkyl, CONH₂, CONHalkyl, CON(alkyl)₂, OH, alkoxy, aryloxy, SH, S-alkyl, S-aryl, SO₂alkyl, SO₃H, SCF₃, CN, halogen, CF₃, NO₂, NH₂, 2°-amino, 3°-amino, NH₂SO₂, OCH₂CH₂NH₂, OCH₂CH₂NHalkyl, OCH₂CH₂N(alkyl)₂, oxazolidin-2-yl, or alkyl substituted oxazolidin-2-yl, with the proviso that R⁴, R⁵, R⁶, R⁷ and R⁸ are not all hydrogen.

32. A composition comprising a compound according to any one of claims 1 to 31 in a pharmaceutical acceptable carrier.

33. The compound of any of claims 1 to 31 or the composition of claim 32 for use as a medicament.

34. The compound of any of claims 1 to 31 or the composition of claim 32 for treating a microbial-caused disease in a patient afflicted therewith.

35. The compound or composition of claim 34 wherein said microbe is a bacterium.

36. The compound or composition of claim 35 wherein said bacterium is a gram positive bacterium.

37. The compound or composition of claim 36, wherein said gram positive bacterium is a member selected from the group consisting of *Staphylococcus* species, *Streptococcus* species, *Bacillus* species, *Mycobacterium* species, *Corynebacterium* species, *Clostridium* species, *Actinomyces* species, *Enterococcus* species, and *Streptomyces* species.

38. The compound or composition of claim 35, wherein said bacterium is a gram negative bacterium.

39. The compound or composition of claim 38, wherein said gram negative bacterium is a member selected from the group consisting of *Acinetobacter* species, *Neisseria* species, *Pseudomonas* species, *Brucella* species, *Agrobacterium* species, *Bordetella* species, *Escherichia species, Shigella* species, *Yersinia* species, *Salmonella* species, *Klebsiella* species, *Enterobacter* species, *Haemophilus* species, *Pasteurella* species, *Streptobacillus* species, spirochetal species, *Campylobacter* species, *Vibrio* species, and *Helicobacter* species.

40. The compound or composition of claim 35, wherein said bacterium is a member selected from the group consisting of *Staphylococcus aureus, Staphylococcus epidermidis, Staphylococcus saprophyticus; Streptococcus pyogenes; Streptococcus agalactiae; Streptococcus pneumoniae; Enterococcus faecalis; Enterococcus faecium; Baccillus anthracis; Mycobacterium avium; Mycobacterium tuberculosis, Acinetobacter baumanii; Corynebacterium diphtheria; Clostridium perfringens; Clostridium botulinum; Clostridium tetani; Neisseria gonorrhoeae; Neisseria meningitidis; Pseudomonos aeruginosa; Legionnella pneumophila; Escherichia coli; Yersinia pestis; Haemophilus influenzae, Helicobacterpylori; Campylobacter fetus; Vibrio cholerae; Vibrio parahemolyticus; Trepomena pallidum; Actinomyces israelii; Rickettsia prowazekii; Rickettsia rickettsii; Chlamydia trachomatis; Chlamydia psittaci; Brucella abortus; Agrobacterium tumefaciens;* and *Francisella tularensis.*

41. The compound of any of claims 1 to 31 or the composition of claim 32 for treating a fungus- or yeast-caused disease in a patient afflicted therewith.
